# EUROPEAN PATENT SPECIFICATION

(11) **EP 3 215 496 B1**
(45) Date of publication and mention of the grant of the patent: **02.01.2019**
(21) Application number: 15828676.5
(22) Date of filing: 06.11.2015
(51) Int. Cl.: C07D 333/24, C07C 323/60, C07C 235/10, C07C 235/34, C07D 213/56, C07D 307/54, A61K 31/164, A61K 31/165, A61K 31/341, A61K 31/381, A61K 31/4402, A61K 31/4406, A61K 31/4409, A61P 31/10, A61P 31/06, A61P 33/02

(54) **PANTOTHENAMIDE ANALOGUES**
PANTOTHENAMIDANALOGA
ANALOGUES DE PANTOTHÉNAMIDE

(30) Priority: 06.11.2014 EP 14192079; 27.08.2015 EP 15182699
(43) Date of publication of application: 13.09.2017
(73) Proprietor: Radboud Universitair Medisch Centrum, 6525 GA Nijmegen (NL); Pansynt B.V., 6525 GA Nijmegen (NL); TropIQ Health Sciences B.V., 6525 GA Nijmegen (NL)
(72) Inventor: HERMKENS, Pedro Harold Han, NL-5345 RC Oss (NL); SCHALKWIJK, Josephus, NL-6584 CK Molenhoek (NL); JANSEN, Patrick Antonius Martinus, NL-6533 XT Nijmegen (NL); BOTMAN, Peter, NL-6605 RM Wijchen (NL)
(74) Representative: Nederlandsch Octrooibureau
(86) International application number: PCT/NL2015/050774
(87) International publication number: WO 2016/072854

(56) References cited:
- WO-A1-2011/152720
- WO-A1-2012/064632
- MARIANNE DE VILLIERS ET AL: "Structural Modification of Pantothenamides Counteracts Degradation by Pantetheinase and Improves Antiplasmodial Activity", ACS MEDICINAL CHEMISTRY LETTERS, vol. 4, no. 8, 8 August 2013 (2013-08-08), pages 784-789, XP055177210, ISSN: 1948-5875, DOI: 10.1021/ml400180d
- AWUAH EMELIA ET AL: "Exploring structural motifs necessary for substrate binding in the active site ofEscherichia colipantothenate kinase", BIOORGANIC & MEDICINAL CHEMISTRY, vol. 22, no. 12, 24 April 2014 (2014-04-24), pages 3083-3090, XP028664747, ISSN: 0968-0896, DOI: 10.1016/J.BMC.2014.04.030
- VIRGA K G ET AL: "Structure-activity relationships and enzyme inhibition of pantothenamide-type pantothenate kinase inhibitors", BIOORGANIC & MEDICINAL CHEMISTRY, PERGAMON, GB, vol. 14, no. 4, 15 February 2006 (2006-02-15), pages 1007-1020, XP027992352, ISSN: 0968-0896 [retrieved on 2006-02-15]
- MERCER A C ET AL: "Antibiotic evaluation and in vivo analysis of alkynyl Coenzyme A antimetabolites in Escherichia coli", BIOORGANIC & MEDICINAL CHEMISTRY LETTERS, PERGAMON, AMSTERDAM, NL, vol. 18, no. 22, 15 November 2008 (2008-11-15), pages 5991-5994, XP025627188, ISSN: 0960-894X, DOI: 10.1016/J.BMCL.2008.07.078 [retrieved on 2008-07-24]
- DATABASE HCAPLUS, [Online] 1 January 1975 (1975-01-01), SARGENT DALE R ET AL: "Antimetabolites of pantothenic acid, ureido- and carbamoyl-derivatives", XP002737435, retrieved from HCAPLUS; STN Database accession no. 1976-517287 & SARGENT D R ET AL: "Antimetabolites of pantothenic acid, ureido- and carbamoyl-derivatives.", TEXAS REPORTS ON BIOLOGY AND MEDICINE 1975, vol. 33, no. 3, 1975, pages 433-443, ISSN: 0040-4675
- JORDAN L. MEIER ET AL: "Synthesis and Evaluation of Bioorthogonal Pantetheine Analogues for in Vivo Protein Modification", JOURNAL OF THE AMERICAN CHEMICAL SOCIETY, vol. 128, no. 37, 1 September 2006 (2006-09-01), pages 12174-12184, XP55024526, ISSN: 0002-7863, DOI: 10.1021/ja063217n

## Description

### FIELD OF THE INVENTION

The present invention concerns compounds and compositions having antimicrobial activity, in particular against bacteria, fungi and/or protozoa. More in particular, the present invention provides novel compounds that are analogues of pantothenamides, which are insensitive to the activity of pantetheinase enzymes and hence are stable in body fluids. The use of these compounds and compositions in the therapeutic and/or prophylactic treatment of microbial infections in humans and animals is also provided.

### BACKGROUND OF THE INVENTION

Pantothenate or pantothenic acid (vitamin B5) is required for CoA biosynthesis and is an essential and rate limiting nutrient for survival and/or growth of numerous bacteria, fungi and protozoa. A range of pantothenate analogues have been reported that possess activity against bacteria, fungi and malaria parasites (for a review see Spry et al 2008, FEMS Microbiol.Rev. 32: 56-106). In 1970, amides derived from pantothenic acid were first reported to possess antibacterial activity *in vitro.* Pantothenamides, of which N-pentylpantothenamide (N5-Pan) and N-heptylpantothenamide (N7-Pan) are the prototypes, are active against Gram-negative and Gram-positive bacteria. During the last few decades, many of these pantothenamides have been synthesized and the putative modes of action have been studied in detail.

Pantothenamides have been shown to serve as substrates or inhibitors (either competitive or allosteric) of pantothenate kinase (PanK), the first enzyme in the CoA biosynthesis pathway. As a consequence PanK-catalysed pantothenate phosphorylation is partially or completely inhibited. In the case that pantothenamides serve as PanK substrates, competing with the natural substrate pantothenic acid, the resulting 4'-phosphopantothenamides may be further metabolized by the CoA biosynthesic machinery to yield analogues of CoA, as was shown for E.coli. Such CoA analogues were found to be incorporated in acyl carrier protein, thereby inhibiting its function in bacterial fatty acid biosynthesis, which requires the 4'-phosphopantetheine moiety of CoA to be active. Whether the mechanisms that ultimately result in antimicrobial activity in the various target organisms (bacteria, fungi, protozoa) are the result of inhibition of CoA biosynthesis, fatty acid biosynthesis or another CoA-utilizing process, or a combination of the above, remains to be resolved.

In spite of their potential use and selectivity for bacterial, fungal and/or protozoan metabolic routes, no pantothenamide compound has ever made it to the clinic.

Jansen et al. (Jansen et al 2013, Antimicrob Agents Chemother 57:4794-4800) have recently shown that pantothenamides are not active as antimicrobials in the presence of serum, and found that they were hydrolyzed by ubiquitous pantetheinases of the vanin family. To address this, a series of pantetheinase inhibitors based on a pantothenate scaffold were synthesized, which proved to inhibit serum pantetheinase activity in the nanomolar range. Mass spectrometric analysis showed that addition of these pantetheinase inhibitors prevented hydrolysis of pantothenamides by serum. Combinations of these novel pantetheinase inhibitors and prototypic pantothenamides like N5-Pan and N7-Pan exert antimicrobial activity *in vitro,* particularly against Gram-positive bacteria (Staphylococcus aureus, Staphylococcus epidermidis, Streptococcus pneumoniae, and Streptococcus pyogenes) even in the presence of serum. These results indicate that pantothenamides, when protected against degradation by host pantetheinases, are potentially useful antimicrobial agents. These findings have also been described in patent applications WO2011/152720 and WO2011/152721 for applications in bacterial and protozoal (malaria) infection.

A related finding was described recently by Spry et al. (Spry et al 2013, Plos One 8: e54974), who studied the effect of a series of pantothenamides on the growth of erythrocytic stage P. falciparum parasites. They found that under standard in vitro culture conditions pantothenamides inhibit parasite growth, albeit with modest potency. They also describe that the antiplasmodial potency of pantothenamides was enhanced considerably when the parasite culture medium used for growth assays (which contained the commonly used serum substitute Albumax II or human serum) is pre-incubated at 37°C for a prolonged period. Consequently, sub-micromolar concentrations of pantothenamides that have no effect in freshly prepared serum-containing medium inhibited parasite growth effectively in the pre-incubated medium. Spry et al. linked this finding to the presence in parasite culture medium of pantetheinase activity, in keeping with the finding described by the above-cited publication by Jansen *et al.* (2013).

Together these data are consistent with pantetheinase-mediated pantothenamide degradation occurring in serum, which forms the likely reason that pantothenamides so far have failed to make it to clinical practice, despite their promising effects on microbial growth and metabolic routes, established *in vitro.*

It is the object of the present invention to provide novel, stable variants of pantothenamides that are active as antimicrobial agents, in particular against bacteria and protozoa, even in the presence of body fluids.

### SUMMARY OF THE INVENTION

The present inventors have surprisingly found that this objective is realized with the pantothenamide analogues described herein.

Compared to the prior art pantothenamide analogues and derivatives, the analogues of this invention are characterized in that, effectively, the amino carboxylate is replaced by a diamino alkyl which can be derivatized with aliphatic or aromatic carbonic acids, via an amide bond. The result is an amide bond in the reversed orientation compared to that of the pantothenamide analogues and derivatives that have been taught by the prior art.

The present inventors established that this 'modification' renders the pantothenamide analogues resistant to pantetheinase hydrolytic activity. This property confers stability in body fluids and makes the compounds suitable for use as antimicrobial agents *in vivo.* This will be illustrated in the appending examples, showing *in vivo* stability and bioavailability of the compounds.

Additionally, the present inventors have discovered that antimicrobial activity of the inverted pantothenamides is preserved compared to the parent compound, although this may vary depending on the compound and the microorganism tested, as will be illustrated in the appending examples.

Pantothenamide analogues have been described in the prior art comprising the modification described above, some of which have been suggested to be pharmacologically active.

For example, WO2012/064632 concerns a broad class of compounds that purportedly act as fatty acid synthase inhibitors. Some of the compounds comprise a moiety corresponding to the 'inverted amide' of this invention but no activity data is provided for any one of the compounds containing that moiety. WO 2003/087040 concerns broad class of compounds that purportedly act as acyl-Coenzyme-A mimics. Some of the compounds comprise a moiety corresponding to the 'inverted amide' of this invention, some of which are shown to affect cholesterol and triglyceride levels in obese rats. WO2014/048547 concerns a broad class of compounds that act as vanin inhibitors. One of the disclosed compounds comprises a moiety corresponding to the 'inverted amide' of the present invention.

None of these documents contains a disclosure of the use of any 'inverted amide' as antimicrobial compound. In fact, the inventors have assessed the capability of compound V of WO2003/087040 and compound 57 of WO2014/048547 to inhibit P. falciparum asexual blood stage replication (using the assay described in example 4 of the experimental part) and established that both were inactive. This finding is in keeping with the Inventor's current beliefs that polarity of substituents in the amide portion of these pantothenamide analogues (inversely) affects this activity.

The present invention thus for the first time provides pantothenamide analogues that are suitable for use in therapeutic and/or prophylactic treatment of microbial infections in a human or animal subject in need thereof.

### DETAILED DESCRIPTION OF THE INVENTION

Hence, a first aspect of the present invention is directed to compounds selected from the group consisting of the pantothenamide analogues represented by formula (I) and pharmaceutically acceptable salts and esters thereof: 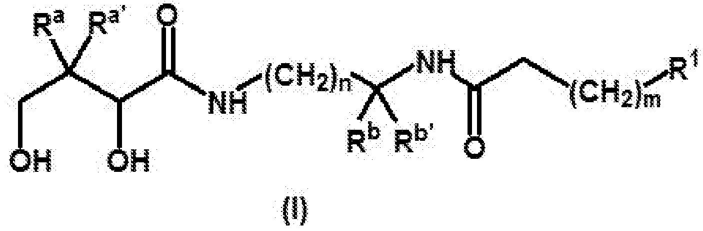 wherein
n = 0, 1,2,3 or 4
m = 0, 1, 2, 3 or 4
R^{a} and R^{a'} are independently selected from the group consisting of (C₁-C₆)alkyl, (C₁-C₆)alkenyl, (C₁-C₆)alkynyl, branched (C₃-C₆)alkyl, O(C₁-C₅)alkyl and CH₂-O-C₁-C₄ alkyl; or R^{a} and R^{a'} are joined together to form a single moiety selected from the group consisting of - (C₃-C₆)alkyl-, -(C₁-C₂)alkyl-O-(C₁-C₂)alkyl- and -(C₁-C₂)alkyl-NR-(C₁-C₂)alkyl-, wherein R represents hydrogen or C₁-C₆ alkyl;
R^{b} and R^{b'} are independently selected from the group consisting of hydrogen, (C₁-C₆)alkyl, (C₁-C₆)alkenyl, (C₁-C₆)alkynyl, branched (C₃-C₆)alkyl, optionally substituted (C₃-C₆)cycloalkyl and optionally substituted aryl; and
R¹ represents hydrogen or an optionally substituted aliphatic, alicyclic, heteroaliphatic, heterocyclic, aromatic or heteroaromatic moiety, said optionally substituted moiety comprising up to 16 carbon atoms.

The compounds of the present invention are referred to herein as 'pantothenamide analogues'. This term is used because the compounds are (inverted amide) analogues of pantothenamide and derivatives thereof, as will be clear to those skilled in the art, based on the explanation and structural formula presented here above.

The compounds of the invention are also referred to herein as 'antimicrobial compounds'. The term 'antimicrobial' means that the compounds have an inhibitory effect on the growth and/or proliferation of at least one microorganism, which can include bacteria, protozoa, fungi, moulds, etc. Hence, the term 'antimicrobial', encompasses more specific terms, such as 'anti-bacterial', 'anti-protozoal', 'anti-fungal', etc. As indicated here above, the compounds of the invention are particularly effective against bacteria and/or protozoa.

In a preferred embodiment of the invention, the optionally substituted aliphatic, alicyclic, heteroaliphatic, heterocyclic, aromatic or heteroaromatic moiety represented by R¹ comprises up to 14, up to 12, up to 10, up to 9, or up to 8 carbon atoms.

In a particularly preferred embodiment R¹ represents
- a saturated or unsaturated, optionally branched and/or substituted, C₁-C₁₀ aliphatic or C₁-C₁₀ heteroaliphatic chain,
- an optionally substituted aryl moiety; or
- an optionally substituted heteroaryl moiety.

In a further preferred embodiment of the invention R¹ represents:
- an aliphatic or heteroaliphatic moiety selected from the group consisting of C₁-C₁₀ alkyl, C₂-C₁₀-alkenyl, C₂-C₁₀-alkynyl, C₁-C₅-alkyl-C₃-C₆ cycloalkyl, (C₁-C_{y})-O-(C₁-Cₓ) and (C₁-C_{y})-S-(C₁-Cₓ), wherein y and x each represent an integer in the range of 0-7 and wherein y+x≤7, each moiety optionally being substituted with 1-3 substituents independently selected from halo, C₁-C₆ alkyl, C₃-C₆ cylcoalkyl, phenyl, C₁-C₆ haloalkyl, C₁-C₆ alkoxy and CH₂-O-C₁-C₆alkyl
- an aromatic moiety selected from the group consisting of phenyl and naphtyl, each optionally substituted with 1-3 substituents independently selected from halo, C₁-C₆ alkyl, C₃-C₆ cylcoalkyl, phenyl, C₁-C₆ haloalkyl, C₁-C₆ alkoxy and CH₂-O-C₁-C₆alkyl;
- a heteroaromatic moiety selected from the group consisting of thienyl, benzothienyl, furyl, benzopyranyl, pyrol, indolyl, pyran, pyrimidine, triazine, pyridyl, quinolyl, isoquinolyl, isoxazole, oxazole, pyrazole, thiazole, imazole, triazole, oxadiazole, thiadiazole and tetrazole, each optionally substituted with 1-3 substituents independently selected from halo, C₁-C₆ alkyl, C₃-C₆ cylcoalkyl, phenyl, C₁-C₆ haloalkyl, C₁-C₆ alkoxy and CH₂-O-C₁-C₆alkyl.

In a preferred embodiment of the invention an antimicrobial compound as defined herein is provided, wherein m=1 or 2 and R¹ represents C₅ or C₆ heteroaryl comprising one heteroatom selected from oxygen, nitrogen and sulphur, optionally substituted with a substituent selected from the group consisting of methyl, ethyl, propyl, t-butyl, methoxy, ethoxy, chloro, fluoro and trifluoromethyl. Although the inventors do not wish to be bound by any particular theory, compounds of the invention wherein R¹ represents heteroaryl have particularly low IC₅₀ values for P. falciparum and are therefore particularly preferred for use as anti-malarial agent and for related applications.

In a preferred embodiment of the invention an antimicrobial compound as defined herein is provided, wherein m=1 or 2 and R¹ represents phenyl, optionally substituted with one or two substituents selected from the group consisting of methyl, ethyl, propyl, t-butyl, methoxy, ethoxy, chloro, fluoro and trifluoromethyl. Although the inventors do not wish to be bound by any particular theory, compounds of the invention wherein R¹ represents phenyl, especially substituted phenyl, combine a low MIC value for bacterial species and low IC₅₀ values for P. falciparum.

In a preferred embodiment of the invention, R^{a} and R^{a'} in formula (I) are independently selected from the group consisting of of (C₁-C₆)alkyl, (C₁-C₆)alkenyl and (C₁-C₆)alkynyl, or R^{a} and R^{a'} are joined together to form a single moiety selected from the group consisting of -(C₃-C₆)alkyl-, -(C₁-C₂)alkyl-O-(C₁-C₂)alkyl- and -(C₁-C₂)alkyl-NH-(C₁-C₂)alkyl-; more preferably from the group consisting of R^{a} and R^{a'} in formula (I) are independently selected from the group consisting of (C₁-C₆)alkyl or are joined together to form a single moiety selected from the group consisting of -(C₃-C₆)alkyl-; more preferably from the group consisting of R^{a} and R^{a'} in formula (I) are independently selected from the group consisting of methyl, ethyl and propyl or are joined together to form a moiety selected from -(CH₂)₅- or -(CH₂)₆- In a particularly preferred embodiment of the invention, at least one of R^{a} and R^{a'} in formula (I) represents methyl. In a most preferred embodiment of the invention R^{a} and R^{a'} in formula (I) both represent methyl.

In a preferred embodiment of the invention, R^{b} and R^{b'} in formula (I) are independently selected from hydrogen, methyl, ethyl, propyl, isopropyl, butyl, *t*-butyl, cyclopropyl, cyclopentyl, cyclohexyl and aryl, more preferably from hydrogen, methyl and ethyl, most preferably from hydrogen and methyl. In a preferred embodiment of the invention, one of R^{b} and R^{b'} represents hydrogen. In a preferred embodiment of the invention, one of R^{b} and R^{b'} represents hydrogen and the other one represents a moiety selected from methyl, ethyl, propyl, isopropyl and cyclopropyl, more preferably from methyl and ethyl, most preferably methyl. In another preferred embodiment of the invention, R^{b} and R^{b'} both represent hydrogen.

In a preferred embodiment of the invention an antimicrobial compound as defined herein is provided, R¹ represents C₁-C₁₀ alkyl. Although the inventors do not wish to be bound by any particular theory, compounds of the invention wherein R¹ represents C₁-C₁₀ alkyl have particularly low MIC values for bacterial species and are therefore particularly preferred for use as anti-bacterial agent and for related applications.

In a preferred embodiment of the invention, n in formula (I) is 1, 2 or 3, more preferably 1 or 2, most preferably 1.

In a preferred embodiment of the invention, m in formula (I) is 1, 2 or 3, more preferably 1 or 2, most preferably 1.

The term "aliphatic", as used herein, includes both saturated and unsaturated, straight chain (i.e., unbranched) or branched aliphatic hydrocarbons as defined by IUPAC, which are optionally substituted with one or more functional groups. As defined herein, "aliphatic" is intended to include optionally substituted alkyl, alkenyl and alkynyl moieties.

The term "heteroaliphatic", as used herein, refers to aliphatic moieties in which one or more carbon atoms in the main chain have been replaced with a heteroatom. Thus, a heteroaliphatic group refers to an aliphatic chain which contains one or more oxygen, sulfur, or nitrogen atoms in place of carbon atoms in the aliphatic main chain.

Aliphatic and heteroaliphatic moieties may be branched or linear unbranched. In certain embodiments, heteroaliphatic moieties are substituted by independent replacement of one or more of the hydrogen atoms thereon.

As utilized herein, the term "alkyl", either alone or within other terms, means an acyclic alkyl radical, preferably containing from 1 to 10, more preferably from 1 to about 8 carbon atoms and most preferably 1 to about 6 carbon atoms. Said alkyl radicals may be linear or branched and may optionally substituted as defined elsewhere in this document. Examples of such radicals include methyl, ethyl, chloroethyl, hydroxyethyl, n-propyl, oxopropyl, isopropyl, n-butyl, cyanobutyl, isobutyl, sec-butyl, tert-butyl, pentyl, aminopentyl, iso-amyl, hexyl, octyl and the like.

The term "alkenyl" refers to an unsaturated, acyclic hydrocarbon radical containing at least one double bond. Such alkenyl radicals typically contain from 2 to 10 carbon atoms, preferably from 2 to 8 carbon atoms and most preferably 2 to about 6 carbon atoms. Said alkenyl radicals may linear or branched and may optionally be substituted as defined elsewhere in this document. Examples of suitable alkenyl radicals include ethenyl, 1-propenyl, 2-propenyl, 2-methyl-1-propenyl, 1-butenyl, 2-butenyl and the like.

The term "alkynyl" refers to an unsaturated, acyclic hydrocarbon radical containing one or more triple bonds, such radicals typically containing from 2 to 10 carbon atoms, preferably having from 2 to 8 carbon atoms and most preferably from 2 to 6 carbon atoms. Said alkynyl radicals may be linear or branched and may optionally be substituted with groups as elsewhere in this document. Examples of suitable alkynyl radicals include ethynyl, propynyl, hydroxypropynyl, butyn-1-yl, butyn-2-yl, pentyn-1-yl, pentyn-2-yl, 4 methoxypentyn-2-yl, 3-methylbutyn-1-yl, hexyn-1-yl, hexyn-2-yl, hexyn-3-yl, 3,3-dimethylbutyn-1-yl radicals and the like.

The term "cycloalkyl" refers to carbocyclic radicals typically having 3 to 10 carbon atoms, preferably 3 to 8 carbon atoms, most preferably 5 to 8 carbon atoms. Said cycloalkyl radicals may optionally be substituted as defined elsewhere in this document. Examples of suitable cycloalkyl radicals include, but are not limited to, cyclopropyl, cyclobutyl, cyclopentyl, cyclohexyl and cycloheptyl.

The term "cycloalkenyl" embraces carbocyclic radicals having 3 to 10 carbon atoms and one or more carbon-carbon double bonds. Preferred cycloalkenyls in accordance with this invention have 3 to 8 carbon atoms, most preferably 5 to 8 carbon atoms. Said cycloalkenyl radicals may optionally be substituted as defined elsewhere in this document. Examples include radicals such as cyclobutenyl, cyclopentenyl, cyclohexenyl and cycloheptenyl.

The term "alicyclic", as used herein, refers to compounds which combine the properties of aliphatic and cyclic compounds and include but are not limited to cyclic, or polycyclic aliphatic hydrocarbons and bridged cycloalkyl compounds, which are optionally substituted with one or more functional groups.

The term "heteroalicyclic", "heterocycloalkyl" or "heterocyclic", as used herein, refers to compounds which combine the properties of heteroaliphatic and cyclic compounds and include but are not limited to saturated and unsaturated mono- or polycyclic ring systems having 5-16 atoms wherein at least one ring atom is a heteroatom selected from O, S and N (wherein the nitrogen and sulfur heteroatoms may be optionally be oxidized), wherein the ring systems are optionally substituted with one or more functional groups, as defined herein.

In general, the term "aromatic moiety", as used herein, refers to a stable mono- or polycyclic, unsaturated moiety having preferably 3-16 carbon atoms, each of which may be substituted or unsubstituted. In certain embodiments, the term "aromatic moiety" refers to a planar ring having p-orbitals perpendicular to the plane of the ring at each ring atom and satisfying the Huckel rule where the number of pi electrons in the ring is (4n+2) wherein n is an integer. A mono- or polycyclic, unsaturated moiety that does not satisfy one or all of these criteria for aromaticity is defined herein as "non-aromatic", and is encompassed by the term "alicyclic". The term "aryl", accordingly, means an aromatic system containing one, two or three rings wherein such rings may be attached together in a pendant manner or may be fused. The term "fused" means that a second ring is present having two adjacent atoms in common with the first ring. The term "fused" is equivalent to the term "condensed". Examples include radicals such as phenyl, naphthyl, tetrahydronaphthyl, indane and biphenyl.

In general, the term "heteroaromatic moiety", as used herein, refers to stable substituted or unsubstituted unsaturated mono-heterocyclic or polyheterocyclic moieties having preferably 3-14 carbon atoms, comprising at least one ring having p-orbitals perpendicular to the plane of the ring at each ring atom, and satisfying the Huckel rule where the number of pi electrons in the ring is (4n+2) wherein n is an integer. The term "heteroaryl", accordingly, means an aromatic ring system containing one, two or three rings, which may be attached in a pendant manner or may be fused, wherein at least one of said rings contains one or more heteroatoms selected from the group consisting of N, O or S. Examples include pyrazole, thiophene, quinazolinyl, oxazole, thiazole, thiadiazole, tetrazole, triazole, imidazole, benzimidazole, thienyl, benzothienyl, furyl, benzopyranyl, pyrol, indolyl, pyran, pyrimidine, triazine, pyridyl, quinolyl, isoquinolyl, isoxazole, imidazole, oxadiazole, dihydroquinolinyl, dihydroquinazolyl and tetrahydroquinazolyl.

Particularly preferred examples of the antimicrobial compounds of the present invention include the compounds denominated CXP18.6-052; CXP18.6-017; CXP18.6-064; CXP 18.6-046; CXP 18.6-047; CXP18.6-018; CXP 18.6-069; CXP 18.6-006; CXP 18.6-043; CXP18.6-026; CXP14.18-019; CXP18.6-028; CXP18.6-042; CXP18.6-012; CXP14.18-038; CXP18.6-057; CXP18.6-019; CXP18.6-056; CXP18.6-008; CXP18.6-027; CXP14.18-034; CXP18.6-013; CXP14.18-010; CXP14.18-008; CXP14.18-016; CXP14.18-028; CXP18.6-022; CXP14.18-037; CXP18.6-014; CXP14.18-035; CXP14.18-005; CXP14.18-014; CXP14.18-007; CXP14.18-012; CXP18.6-055; CXP14.18-020; CXP18.6-048; CXP18.6-007; CXP18.6-038; CXP18.6-009; CXP18.6-010, MMV689258; and MMV689260, having the structures as shown in the Table 1, as well as the pharmaceutically acceptable salts and esters thereof.

**Table 1: Examples of compounds with IC₅₀ (for P.falciparum) and MIC (for bacteria).**

| Compound | Structure | IC₅₀ (µM) P.falciparum | MIC (µg/ml) for most sensitive bacterial species |
|---|---|---|---|
| CXP18.6-052 | 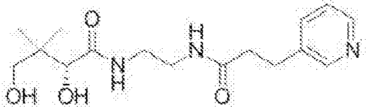 | 0.008 | >32* |
| CXP18.6-017 | 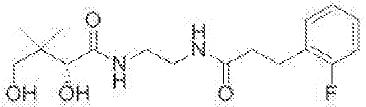 | 0.022 | 2 (SA/SE) |
| CXP18.6-064 | 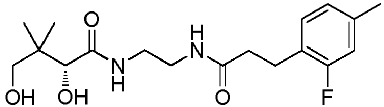 | 0.042 | 2 (SE)* |
| CXP18.6-046 | 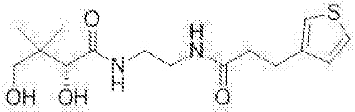 | 0.057 | 32 (SP) |
| CXP18.6-047 | 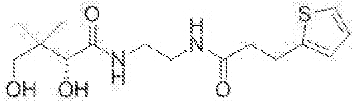 | 0.1 | 8 (SP) |
| CXP18.6-018 | 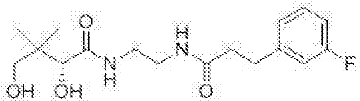 | 0.1 | 16 (SP) |
| CXP18.6-069 | 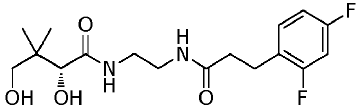 | 0.11 | 4 (SE)* |
| CXP18.6-006 | 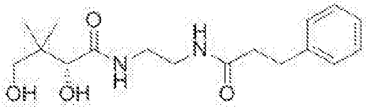 | 0.12 | 16 (SE) |
| CXP18.6-043 | 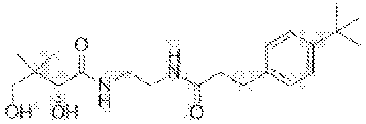 | 0.15 | >32 |
| CXP18.6-026 | 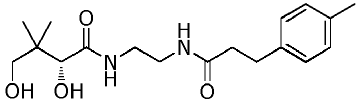 | 0.16 | >32 |
| CXP14.18-019 | 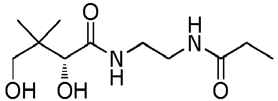 | 0.17 | >32* |
| CXP18.6-028 | 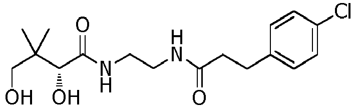 | 0.19 | >32 |
| CXP18.6-042 | 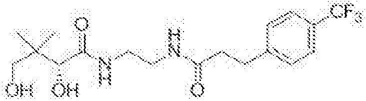 | 0.21 | >32 |
| CXP18.6-012 | 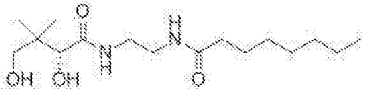 | 0.34 | 1 (SA) |
| CXP14.18-038 | 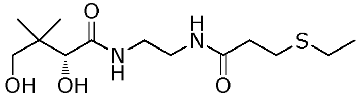 | 0.37 | 8 (SP)* |
| CXP18.6-057 | 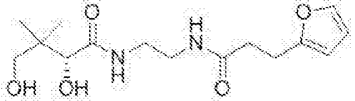 | 0.4 | 16 (SA/SE)* |
| CXP18.6-019 | 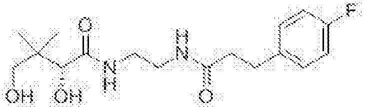 | 0.42 | 32 (SE) |
| CXP18.6-056 | 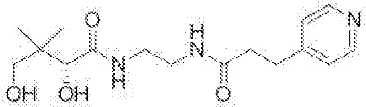 | 0.47 | >32* |
| CXP18.6-008 | 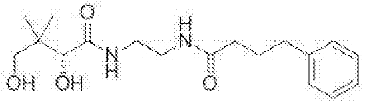 | 0.57 | 16 (SE) |
| CXP18.6-027 | 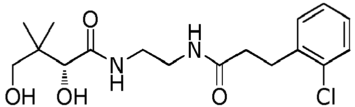 | 0.58 | >32 |
| CXP14.18-034 | 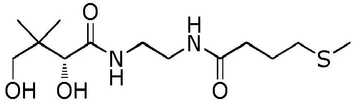 | 0.62 | 8 (SP)* |
| CXP18.6-013 | 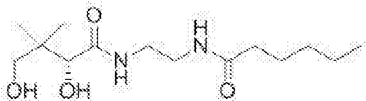 | 0.63 | 1 (SE), 4 (SPn) |
| CXP14.18-010 | 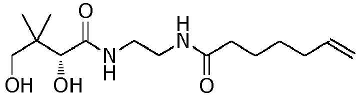 | 0.71 | 16 (SP)* |
| CXP14.18-008 | 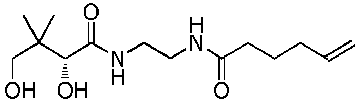 | 0.74 | 16 (SP) |
| CXP14.18-016 | 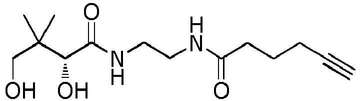 | 0.81 | 32 (SP) |
| CXP14.18-028 | 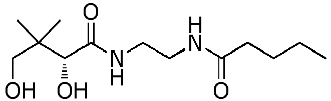 | 0.85 | 2 (SP)* |
| CXP18.6-022 | 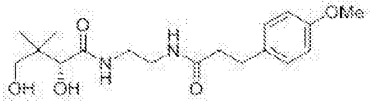 | 1 | >32 |
| CXP14.18-037 | 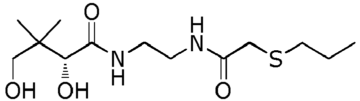 | 1.34 | 4 (SP)* |
| CXP18.6-014 | 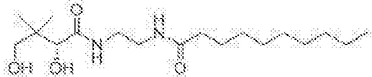 | 7.5 | 1 (SA/SE) |
| CXP14.18-035 | 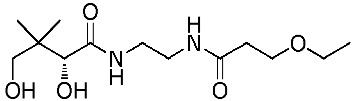 | >10 | 16 (SP)* |
| CXP14.18-005 | 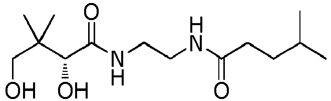 | 2.78 | 8 (SP) |
| CXP14.18-014 | 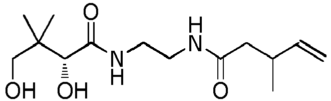 | 4.93 | 16 (SP) |
| CXP14.18-007 | 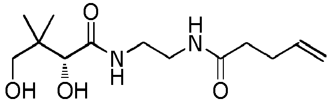 | 5.15 | 16 (SP) |
| CXP14.18-012 | 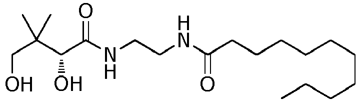 | >10 | 8 (SE) |
| CXP18.6-055 | 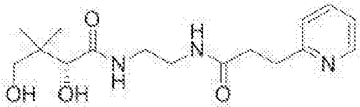 | 1.6 | 32 (SE)* |
| CXP14.18-020 | 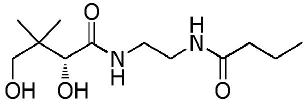 | 2.0 | 32 (SP)* |
| CXP18.6-048 | 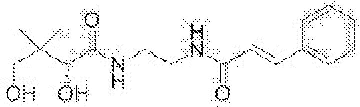 | 2.2 | 16 (SE) |
| CXP18.6-007 | 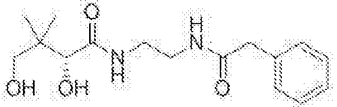 | 3.9 | 16 (SP) |
| CXP18.6-038 | 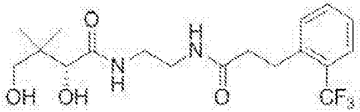 | 6.4 | 32 (SE) |
| CXP 18.6-009 | 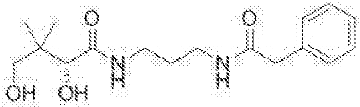 | 9.2 | >32 |
| CXP18.6-010 | 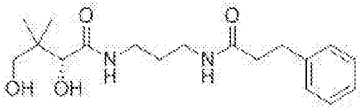 | 7.5 | >32 |
| MMV689258 | 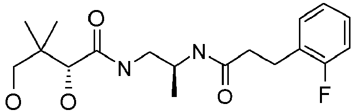 | 0.004 | >32 |
| MMV689260 | 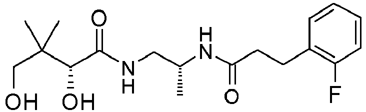 | 0.21 | >32 |

*The IC50 (µM) and MIC (µg*/*ml) values were determined as indicated in the Examples section. All compounds were tested for Saureus (SA), S.epidermidis (SE), S.pyogenes (SP), and S.pneumoniae (SPn) except for compounds marked with* *, *which were not tested for S.pneumoniae.*

Compounds of the invention can contain one or more chiral centers and/or double bonds and, therefore, exist as stereoisomers, such as double-bond isomers (i.e., geometric isomers), enantiomers, or diastereomers. According to the invention, the chemical structures depicted herein, and therefore the compounds of the invention, encompass all of the corresponding compound's enantiomers and stereoisomers, that is, both the stereomerically pure form (e.g., geometrically pure, enantiomerically pure, or diastereomerically pure) and enantiomeric and stereoisomeric mixtures.

A compound of the invention is considered optically active or enantiomerically pure (i.e., substantially the R-form or substantially the S-form) with respect to a chiral center when the compound is about 90% enantiomeric excess (ee) or greater, preferably, equal to or greater than 95% enantiomeric excess with respect to a particular chiral center. A compound of the invention is considered to be in enantiomerically-enriched form when the compound has an enantiomeric excess of greater than about 1% ee, preferably greater than about 5% ee, more preferably, greater than about 10% ee with respect to a particular chiral center. A compound of the invention is considered diastereomerically pure with respect to multiple chiral centers when the compound is about 90% de (diastereomeric excess) or greater, preferably, equal to or greater than 95% de with respect to a particular chiral center. A compound of the invention is considered to be in diastereomerically-enriched form when the compound has an diastereomeric excess of greater than about 1% de, preferably greater than about 5% de, more preferably, greater than about 10% de with respect to a particular chiral center. As used herein, a racemic mixture means about 50% of one enantiomer and about 50% of is corresponding enantiomer relative to all chiral centers in the molecule. Thus, the invention encompasses all enantiomerically-pure, enantiomerically-enriched, diastereomerically pure, diastereomerically enriched, and racemic mixtures of compounds of Formula I.

Enantiomeric and diastereomeric mixtures can be resolved into their component enantiomers or stereoisomers by well known methods, such as chiral-phase gas chromatography, chiral-phase high performance liquid chromatography, crystallizing the compound as a chiral salt complex, or crystallizing the compound in a chiral solvent. Enantiomers and diastereomers can also be obtained from diastereomerically- or enantiomerically-pure intermediates, reagents, and catalysts by well known asymmetric synthetic methods.

In a particularly preferred embodiment of the invention, the pantothenamide analogues are 'enantiomerically pure' (i.e. according to the above definitions) derivatives or analogues of D(+)-pantothenamide, i.e. 'enantiomerically pure' substances possessing the same stereochemical arrangement as the corresponding stereocenter in D(+)- pantothenamide.

The compounds and combinations may be used pharmaceutically in the form of the free base, in the form of salts, solvates and as hydrates. All forms are within the scope of the invention. The term "pharmaceutically acceptable salts, esters, and prodrugs" as used herein refers to salts, amino acid addition salts, esters, and prodrugs of the compounds of the present invention which are, within the scope of sound medical judgment, suitable for use in contact with the tissues of patients without undue toxicity, irritation, allergic response, and the like, commensurate with a reasonable benefit/risk ratio, and effective for their intended use, as well as the zwitterionic forms, where possible, of the compounds of the invention.

The term "salts" refers to inorganic and organic acid or base addition salts of compounds of the present invention. These salts can be prepared *in situ* during the final isolation and purification of the compounds or by separately reacting the purified compound in its free base form with a suitable organic or inorganic acid and isolating the salt thus formed. Depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge et al., "Pharmaceutical Salts", Journal of Pharmaceutical Science, 1977, 66, 1-19). Acid and basic addition salts may be formed with the compounds of the invention for use as sources of the free base form even if the particular salt per se is desired only as an intermediate product as, for example, when the salt is formed only for the purposes of purification and identification.

Examples of suitable esters include compounds of the invention which hydrolyze *in vivo* and include those that break down readily in the human body to leave the parent compound or a salt thereof. Examples of pharmaceutically acceptable, relatively non-toxic esters of the invention include C₁-C₆ alkyl esters and C₅-C₇ cycloalkyl esters. Esters of the compounds of the invention can be prepared according to conventional methods. Pharmaceutically acceptable esters can be obtained through reaction of hydroxy groups of the compound with an organic acid, such as acetic acid or benzoic acid. In the case of compounds containing carboxylic acid groups, the pharmaceutically acceptable esters are prepared by reaction of said carboxylic acid group, as will be understood by those skilled in the art.

Furthermore, the compounds of the present invention can exist in unsolvated as well as solvated forms with pharmaceutically acceptable solvents such as water, ethanol, and the like. In general, the solvated forms are considered equivalent to the unsolvated forms for the purposes of the present invention.

The compounds of the invention can be provided as crystalline or amorphous products. They may be obtained, for example, as solid plugs, powders, or films by methods such as precipitation, crystallization, freeze drying, spray drying, or evaporative drying. Microwave or radio frequency drying may be used for this purpose.

Other aspect of the present invention concerns the pantothenamide analogues of the invention as defined herein before, for use as a medicament for treating a human or animal subject in need thereof and a method of therapeutic and/or prophylactic treatment of a human or animal subject in need thereof, said method comprising administering an effective amount of a pantothenamide analogue of the invention.

Typically these methods and uses concern the treatment or prevention of an microbial infection in a human or animal subject in need thereof, more preferably the treatment and/or prevention of a bacterial infection, a fungal or yeast infection, or a protozoan infection.

In another embodiment, the methods and uses as defined here concern the prevention and/or treatment of disease caused by bacteria, fungus, yeast or protozoa.

In a particularly preferred embodiment of the invention, the compounds for use in the methods and uses as defined here concern the compounds of the invention for use in the prevention and/or treatment of infection by a protistan parasite of the genus Plasmodium, Trypanosoma, Giardia, Cryptosporidium, Amoeba, Toxoplasma, Trichomonas or Leishmania, more preferably a protistan parasite selected from the group consisting of P. falciparum, P. vivax, P. malariae, P. ovale and/or a disease caused by said protozoa. In one embodiment, said disease is a disease selected from the group consisting of Malaria, Amoebiasis, Giardiasis, Toxoplasmosis, Cryptosporidiosis, Trichomoniasis, Chagas disease, Leishmaniasis, Sleeping Sickness, Dysentery, Acanthamoeba Keratitis, Primary Amoebic Meningoencephalitis.

In a particularly preferred embodiment of the invention, the methods and uses as defined here concern the prevention and/or treatment of infection by bacteria, more preferably bacteria selected from the group of gram-positive bacteria, especially gram-positive bacteria selected from staphylococci spp. and streptococci spp., such as S.aureus, S.epidermidis, S.pneumoniae and S.pyogenes.

In a preferred embodiment of the invention, these uses and methods concern treatment of human subjects in need thereof, especially a subject infected by or at risk of becoming infected by bacteria, fungi, yeast or protozoa as defined here above and/or a subject at risk of attracting a disease or condition as defined herein.

As used herein, and as well understood in the art, "treatment" is an approach for obtaining beneficial or desired results, including clinical results. Beneficial or desired clinical results can include, but are not limited to, alleviation or amelioration of one or more symptoms or conditions, diminishment of extent of disease, stabilized (i.e. not worsening) state of disease, preventing spread of disease, delay or slowing of disease progression, amelioration or palliation of the disease state, and remission. "Treatment" can also mean prolonging survival as compared to expected survival if not receiving treatment. The term "prevention" or "prophylaxis", or synonym thereto, as used herein refers to a reduction in the risk or probability of a patient attracting an infection or disease associated with infection.

The term a "therapeutically effective amount", "effective amount" or a "sufficient amount" of a compound of the present invention is a quantity sufficient to, when administered to the subject, including a mammal, for example a human, effect beneficial or desired results, including clinical results, and, as such, an "effective amount" or synonym thereto depends upon the context in which it is being applied. In the context of disease, therapeutically effective amounts of the compounds of the present invention are used to treat, modulate, attenuate, reverse, or effect microbial infection in a mammal. An "effective amount" is intended to mean that amount of a compound that is sufficient to treat, prevent or inhibit microbial infections and/or diseases associated with such microbial infections.

In certain embodiments, the disease or disorder associated with microbial infections is malaria. Malaria is caused by a Plasmodium parasite, in particular P. falciparum, P. vivax, P. ovale or P. malariae. Thus an effective amount is the amount sufficient to, when administered to the human or animal subject, prevent or inhibit malaria or a disease or a disorder associated with malaria or infection with a malaria parasite. The amount of a given compound of the present invention that will correspond to such an amount will vary depending upon various factors, such as the given drug or compound, the pharmaceutical formulation, the route of administration, the type of disease or disorder, the identity of the subject or host being treated, and the like, but can nevertheless be routinely determined by one skilled in the art. Also, as used herein, a "therapeutically effective amount" of a compound of the present invention is an amount which prevents, inhibits, suppresses or reduces malaria, e.g., as determined by clinical symptoms such as fever, anemia, and in severe cases, a coma potentially leading to death.

For administration to human patients, the total daily dose of the pantothenamide analogue of the invention is typically within the range 0.0001 mg/kg to 500 mg/kg body weight, preferably 0.001 mg/kg to 250 mg/kg body weight, more preferably 0.005 mg/kg to 50 mg/kg body weight, most preferably 0.01 mg/kg to 10 mg/kg body weight, the exact amount depending of course on the mode of administration and/or the severity of the disease or condition. For example, daily dosages for treatment by intravenous administration will typically be much lower than for oral treatment.

It is preferred that the pantothenamide analogues of the invention are administered repeatedly. Preferably, the compound is administered once, twice or three times daily to the patient. Embodiments are also envisaged wherein the compound of the present invention is administered less than once daily, e.g. once every two days, once every three days, once every four days or once a week. Even less frequent administration may be feasible using depot formulations.

Treatment may commence before, during or after exposure of the subject to a (potentially) infectious microorganism. The length of the treatment period depends on a variety of factors, such as the severity of the infection and/or disease, the age of the patient, the concentration and the activity of the pantothenamide analogue used. Typically, treatment lasts at least one day, more preferably at least two days, more preferably one week, more preferably at least two weeks, more preferably at least three weeks. As is generally known by those skilled in the art, repeated and continued administration typically reduces the risks of development of resistance towards the antimicrobial and, without wishing to be bound by any particular theory, it is hypothesized that this might apply to compounds and combinations of the present invention.

It will also be appreciated that the effective dosage of the compound used for the treatment or prophylaxis may increase or decrease over the course of a particular treatment or prophylaxis regime. Changes in dosage may result and become apparent by standard diagnostic assays known in the art. In some instances, chronic administration may be required.

The pantothenamide analogues of the invention can be administered through any of the conventional routes. Oral administration may involve swallowing, so that the compound enters the gastrointestinal tract, or buccal or sublingual administration may be employed by which the compound enters the blood stream directly from the mouth. Parenteral administration may involve administration directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques. The compounds of the invention may furthermore be administered transdermally, topically, intranasally or pulmonally. It is preferred that the pantothenamide analogues of the invention are administered orally, parenterally or topically, preferably orally or intravenously.

Pantothenamide analogues of the invention may typically be administered to a human or animal subject as a pharmaceutical composition that is designed and optimized for a particular route of administration. Hence, another aspect of the invention concerns pharmaceutical compositions comprising pantothenamide analogue according to the invention.

Generally, the pantothenamide analogues will be administered as a formulation in association with one or more pharmaceutically acceptable excipients. The term "excipient" as used herein refers to any ingredient other than the pantothenamide analogue of the invention. The choice of excipient will, to a large extent, depend on factors such as the particular mode of administration, the effect of the excipient on solubility and stability, and the nature of the dosage form.

In a preferred embodiment of the invention a pharmaceutical composition for use in humans is provided comprising a pantothenamide analogue of the invention in a total amount within the range of 0.001 mg to 1000 mg, preferably 0.01 mg to 250 mg, more preferably 0.05 mg to 100 mg, most preferably 0.1 to 50 mg.

Compositions suitable for the delivery of the pantothenamide analogues of the present invention and methods for their preparation will be readily apparent to those skilled in the art. Such compositions and methods for their preparation may be found, for example, in 'Remington's Pharmaceutical Sciences', 19th Edition (Mack Publishing Company, 1995).

Particularly suitable formulations for oral therapeutic administration, include tablets, buccal tablets, troches, capsules, elixirs, suspensions, syrups, wafers, and the like. Formulations suitable for oral administration include solid formulations such as tablets, capsules containing particulates, liquids, or powders, lozenges (including liquid-filled), chews, multi- and nano- particulates, gels, solid solution, liposome, films, ovules, sprays and liquid formulations.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The pantothenamide analogues of the invention may also be incorporated in fast-dissolving, fast-disintegrating dosage forms such as those described in Expert Opinion in Therapeutic Patents, 1-1 (6), 981-986, by Liang and Chen (2001).

For tablet dosage forms, depending on dose, the pantothenamide analogue may make up from 1 weight % to 80 weight % of the dosage form, more typically from 5 weight % to 60 weight % of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl -substituted hydroxypropyl cellulose, starch, pregelatinised starch and sodium alginate. Generally, the disintegrant will comprise from 1 weight % to 25 weight %, preferably from 5 weight % to 20 weight % of the dosage form. Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate. Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet. Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet. Other possible ingredients include anti-oxidants, colourants, flavouring agents, preservatives and taste- masking agents. Exemplary tablets contain up to about 80% drug, from about 10 weight % to about 90 weight % binder, from about 0 weight % to about 85 weight % diluent, from about 2 weight % to about 10 weight % disintegrant, and from about 0.25 weight % to about 10 weight % lubricant. Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated. Various other materials may be present as coatings or to otherwise modify the physical form of the dosage unit. For instance, tablets may be coated with shellac, sugar or both. A syrup or elixir may contain the active compounds sucrose as a sweetening agent, methyl and propylparabens as preservatives, a dye and flavoring, such as cherry or orange flavor. Of course, any material used in preparing any dosage unit form should be pharmaceutically pure and substantially non-toxic in the amounts employed. In addition, the active compounds may be incorporated into sustained-release preparation and formulations. The formulation of tablets is discussed in Pharmaceutical Dosage Forms: Tablets, Vol. 1, by H. Lieberman and L. Lachman (Marcel Dekker, New York, 1980). Suitable modified release formulations for the purposes of the invention are, such as high energy dispersions and osmotic and coated particles, are to be found in Pharmaceutical Technology On-line, 25(2), 1-14, by Verma et al (2001). The use of chewing gum to achieve controlled release is described in WO 00/35298.

A pantothenamide analogue of the invention may also be administered parenterally. Solutions of a compound of the invention can be prepared in water suitably mixed with a surfactant such as hydroxypropylcellulose. Dispersions can also be prepared in glycerol, liquid polyethylene glycols, DMSO and mixtures thereof with or without alcohol, and in oils. Under ordinary conditions of storage and use, these preparations contain a preservative to prevent the growth of microorganisms. A person skilled in the art would know how to prepare suitable formulations. Conventional procedures and ingredients for the selection and preparation of suitable formulations are described, for example, in Remington's Pharmaceutical Sciences (2003-20th edition) and in The United States Pharmacopeia: The National Formulary (USP 24 NF19) published in 1999. The pharmaceutical forms suitable for injectable use include aqueous solutions or dispersions as well as powders for the extemporaneous preparation of injectable solutions or dispersions. In all cases the form must be sterile. Furthermore the final injectable must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin. Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water. The preparation of parenteral formulations under sterile conditions, for example, by lyophilisation, may readily be accomplished using standard pharmaceutical techniques well known to those skilled in the art. The solubility of compounds used in the preparation of parenteral solutions may be increased by the use of appropriate formulation techniques, such as the incorporation of solubility- enhancing agents. Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLA microspheres.

A pantothenamide analogue of the invention may also be administered topically, e.g. to the skin, the eye and/or mucosa. Hence, the pharmaceutical composition may be a suspension, solution, ointment, lotion, cream, foam, spray, balm or patch or occlusion bandage comprising a solution and/or suspension comprising the pantothenamide analogue, etc. Preferably however, the pharmaceutical composition may be selected from the group consisting of a lotion, an ointment, a gel, a cream and a spray. Solutions, creams, ointments or gels according to the present invention are semi-solid formulations that may be made by mixing the pantothenamide analogue, either as a finely divided or powdered form or in solution or suspension in an aqueous or non-aqueous fluid, with a greasy or non-greasy base, such as known to the person skilled in the art. Examples of bases are bases that may comprise one or more hydrocarbons such as hard, soft or liquid paraffin, glycerol, paraffin oil, beeswax, a metallic soap; a mucilage; an oil of natural origin such as almond, corn, arachis, castor or olive oil or derivatives thereof such as castor oil polyoxyl; wool fat or its derivatives or a fatty acid and/or ester such as steric or oleic acid, or isopropyl myristate. The base may furthermore comprise an alcohol such as propylene glycol, polyethylene glycol (PEG) of different molecular weights, cetyl alcohol, ethanol or a macrogel. The formulation may incorporate any suitable surface active agent or emulsifier such as an anionic, cationic or nonionic surfactant such as a sorbitan ester, polysorbate, Cremophor EL, Tween 20, or a polyoxyethylene derivative thereof. Suspending agents such as natural gums, cellulose derivatives or inorganic materials such as silicaceous silicas, and other ingredients such as lanolin, may also be included. An eye lotion may comprise a sterile aqueous solution and may be prepared by standard methods. Lotions or liniments for application to the skin may also include an agent to hasten drying and to cool the skin, such as an alcohol or acetone, and/or a moisturizer such as glycerol or an oil such as castor oil or arachis oil. In one preferred embodiment, the pharmaceutical formulations according to the present invention comprise one or more compounds selected from the group consisting of emulsifiers, hydroxy compounds and lipids, known to the person skilled in the art to be suitable for pharmaceutical formulations for topical administration. Preferably, the emulsifier is selected from the group consisting of Cremophor EL, Tween 20, polysorbate 80 and mixtures thereof, more preferably, the emulsifier is polysorbate 80. Preferably, the hydroxy compound is selected from the group consisting of ethanol, glycerol, propylene glycol, polyethylene glycol (PEG), cetyl alcohol and mixtures thereof. PEG-may be any molecular weight PEG, preferably however PEG 6000. Preferably, the lipid is selected from the group consisting of fatty alcohols, fatty acid esters, mineral oil, oil of natural origin and derivatives thereof and mixtures thereof. More preferably the lipid is selected from the group consisting of Castor oil polyoxyl, paraffin oil and isopropyl myristate.

The pantothenamide analogues of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration. Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, i.e. as a carrier, diluent, or solubiliser.

As will be understood by those skilled in the art, the present invention provides compositions comprising combinations of two or more pantothenamide analogues of the invention.

The pantothenamide analogues of this invention can also be employed in conjunction with other active ingredients conventionally employed in the treatment of the conditions mentioned above. Such combined treatment may result in further enhancement of the efficacy of the treatment. Such further enhancement may be additive or even synergistic.

Suitable examples of other active ingredients that can suitably be combined with the pantothenamide analogues of this invention include other antimalaria agents, such as Atovaquone, Chloroquine, Hydroxychloroquine, Primaquine, Proguanil, Quinidine, Quinine, Sulfadoxine and Pyrimethamine, mefloquine, piperaquine, pyronaridine, artemisinin, artesunate, artemether and artenimol. One example of new compounds invented to use for the treatment of malaria is the class of phenazines, especially riminophenazines, which have a substituted imino group in one benzene ring. In particular, N,5-bis-(phenyl)-3,5-dihydro-3-(cyclohexylimino)-2-phenazinamine has been reported to show antimalarial activity. As will be understood by those skilled in the art, combinations of a pantothenamide analogue of the invention and an antimalaria agent, is typically used in methods of treating and/or preventing malaria, and/or related conditions, typically by co-administration of the pantothenamide analogue and the antimalaria agent.

According to another embodiment of the present invention, the pantothenamide analogues defined herein, can also be used in combination with one or more additional antimicrobial or antibacterial agents from a different class. Such combinations may result in an antimicrobial product with an increased spectrum of action and restored efficacy against resistant bacteria. Suitable examples of such other (classes of) therapeutic agents which may be used in combination with the pantothenamide analogues of the invention include, but are by no means limited to:
- aminoglycosides such as netilmicin, kanamycin, gentamycin, streptomycin, amikacin, and tobramycin;
- macrolides such as erythromycin and lincomycin;
- tetracyclines such as tetracycline, doxycycline, chlortetracycline, and minocycline;
- oxalidinones such as linezoloid; and fusidic acid; and chloramphenicol.
- beta-lactam penicillins such as penicillin, dicloxacillin, and ampicillin;
- beta lactam cephalsporins such as cefepime, ceftazidime, cefotaxime, cefuroxime, cefaclor, and cetriaxone;
- beta lactam carbapenems such as imipenem and meropenem;
- quinolones such as ciprofloxacin, moxifloxacin, and levofloxacin;
- sulfonamides such as sulfanilamide and sulfamethoxazole; metronidazole; rifampin; vancomycin; and nitrofurantoin
- bacteriocins, such as agrocin alveicin carnocin colicin curvaticin divercin enterocin enterolysin epidermin erwiniocin glycinecin halocin lactococin lacticin leucoccin mesentericin nisin pediocin plantaricin sakacin subtilin sulfolobicin vibriocin warnerin

As will be understood by those skilled in the art, combinations of a pantothenamide analogue of the invention and an antibacterial agent, is typically used in methods of treating and/or preventing bacterial infections, typically by co-administration of the pantothenamide analogue and the antibacterial agent.

According to another embodiment of the present invention, the pantothenamide analogues defined herein, can also be used in combination with one or more antiinflammatory agents. Such combinations are, for example, particularly suitable in case the pharmaceutical formulation is a formulation for topical application. Suitable examples of antiinflammatory agents include, but are not limited to:
- NSAIDs, such as salicicylate; acetylsalicylate; Diflunisal; Salsalate; Choline Magnesium Trisalicylate; Ibuprofen; Dexibuprofen; Naproxen; Fenoprofen; Ketoprofen; Dexketoprofen; Flurbiprofen; Oxaprozin; Loxoprofen; Indomethacin; Tolmetin; Sulindac; Etodolac; Ketorolac; Diclofenac; Aceclofenac; Nabumetone; Piroxicam; Meloxicam; Tenoxicam; Droxicam; Lornoxicam; Isoxicam; Mefenamic acid; Meclofenamic acid; Flufenamic acid; Tolfenamic acid; Sulfonanilide; Nimesulide; Licofelone; arylalkanoic acid, 2-arylpropionic acid, N-arylanthranilic acid and oxiam;
- COX-2 inhibitors, such as rofecoxib, valdecoxib, parecoxib, etoricoxib, lumiracoxib, etoriocoxib, firocoxib and celecoxib;
- corticosteroids, such as betamethasone, cortisol, cortisone, dexamethasone, beclometasone, hydrocortisone, methylprednisolone, prednisolone, prednisone, fludrocortisones, deoxycorticosterone, aldosterone and triamcinolone
- Calcineurin inhibitors such as ciclosporin A, tacrolimus and pimecrolimus.
- Coal tar based preparations such as pix lithantracis and liquor carbonis detergens

Another class of therapeutic agents which may suitably be used in conjunction with the pantothenamide analogues of the invention include the so-called resistance modifying agents. Resistance modifying agents may target and inhibit multiple drug resistance (MDR) mechanisms, rendering the parasites or bacteria susceptible to antimicrobials to which they were previously resistant. These compounds include among others efflux inhibitors and Beta Lactamase inhibitors.

As used herein, the terms "co-administration", "co-administered" and "in combination with", referring to the compounds of the invention and, optionally, the one or more other therapeutic agents, is intended to mean, and does refer to and include the following:
- simultaneous administration of such combination of compounds, when such components are formulated together into a single dosage form which releases said components at substantially the same time following administration,
- substantially simultaneous administration of such combination of compounds, when such components are formulated apart from each other into separate dosage forms which are administered at substantially the same time, where after said components are released at substantially the same time,
- sequential administration of such combination of compounds, when such components are formulated apart from each other into separate dosage forms which are administered at consecutive times with a significant time interval between each administration; and

It is within the scope of the present invention that two or more pharmaceutical compositions, at least one of which contains a pantothenamide analogue in accordance with the invention, may conveniently be combined in the form of a kit suitable for co-administration of the compositions. Thus a kit of the invention typically comprises two or more separate pharmaceutical or veterinary composition, at least one of which contains a pantothenamide analogue in accordance with the invention as well as means for separately retaining said composition, such as a container, divided bottle, or divided foil packet. An example of such a kit is the familiar blister pack used for the packaging of tablets, capsules and the like.

Furthermore, for a proper understanding of this document and in its claims, it is to be understood that the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

The following examples are offered for illustrative purposes only, and are not intended to limit the scope of the present invention in any way

### DESCRIPTION OF THE FIGURES

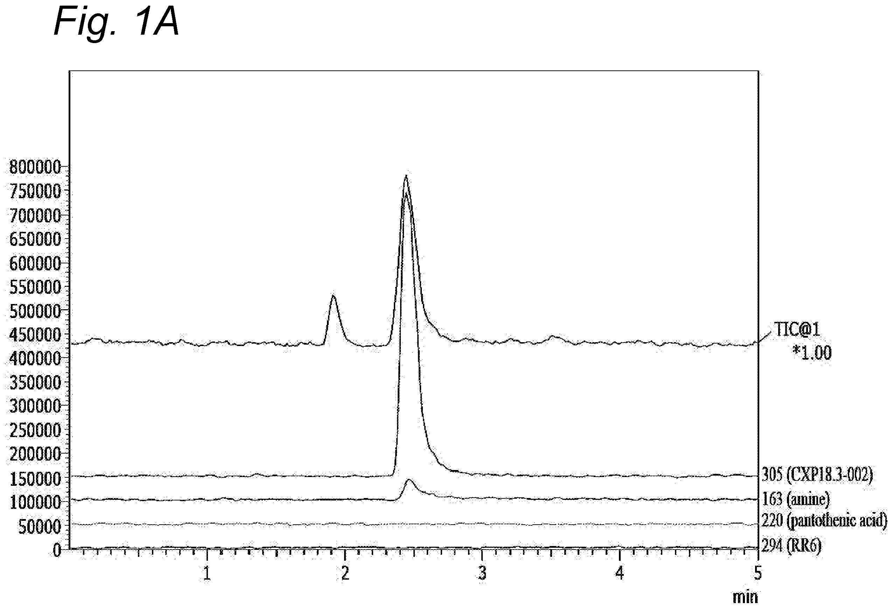

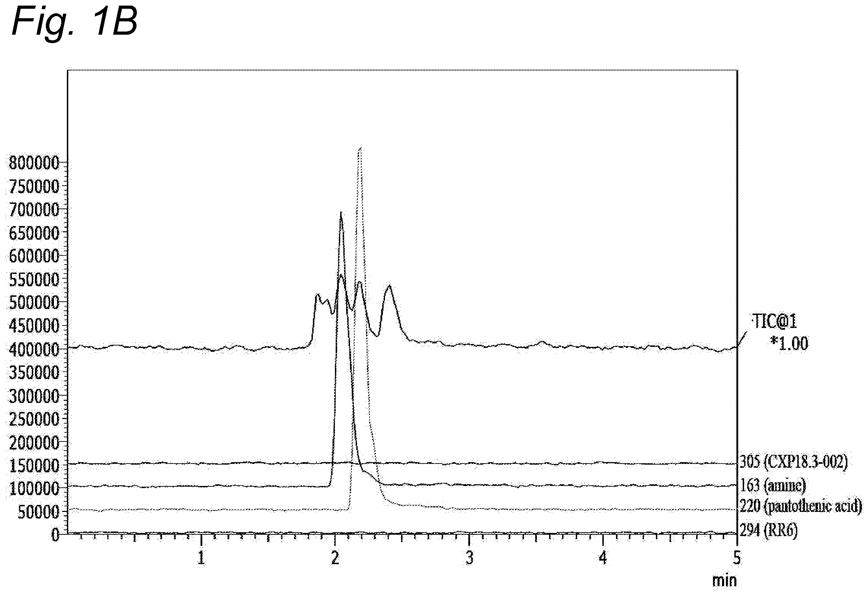

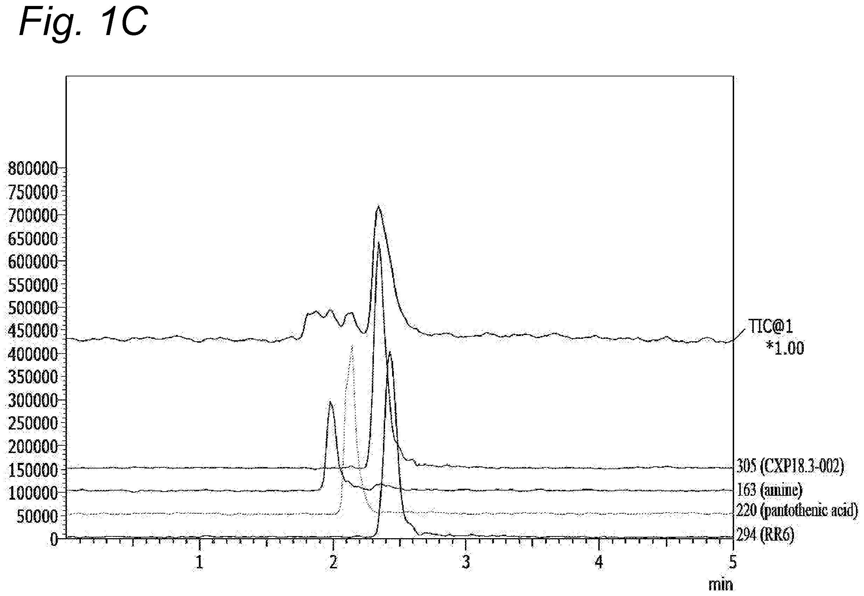

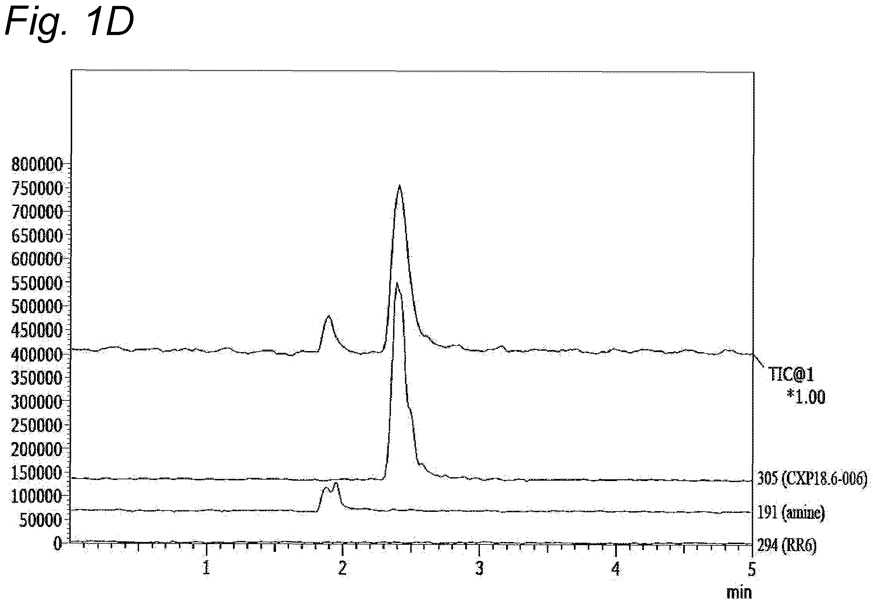

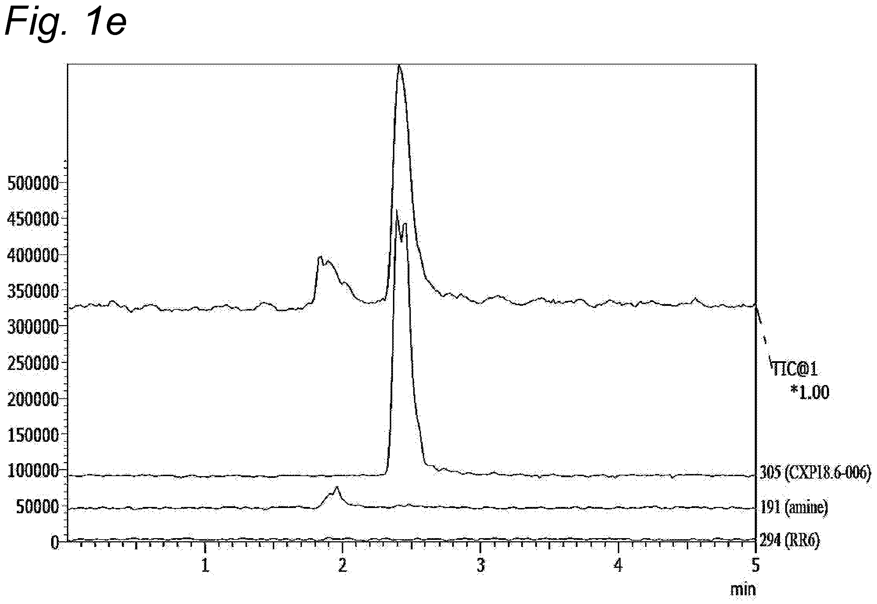

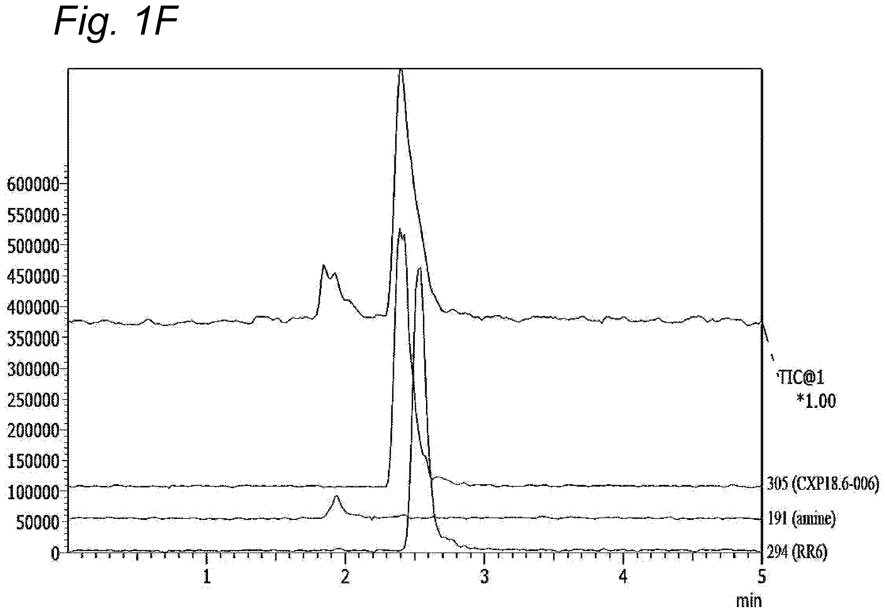

**Fig. 1****:** Inverted amides are stable in serum. The stability of pantothenamide CXP18.3-002 (A-C) and its corresponding inverted amide CXP18.6-006 (D-F) was measured using LC-MS analysis after overnight incubation in buffer (A and D), buffer with 10% fetal bovine serum (B and E) and buffer with 10% FBS supplemented with the vanin inhibitor RR6 (C-F). Fetal bovine serum contains a high level of vanin (pantetheinase) activity. Both compounds are stable after incubation in buffer alone (figure A and D). The small peaks in the amine channels, with molecular masses of 163 (A) en 191 (D), are either the result of disruption of compound during ionization (CXP18.3-002-sample) or background from the buffer (CXP18.6-006-sample). Overnight incubation in 10% serum resulted in complete degradation of CXP18.3-002 resulting in pantothenic acid (and the liberated amine in figure B. CXP18.6-006 remains intact in serum-containing buffer, and no amine is formed in figure E. Note that the hypothetical hydrolysis product phenylpropionic acid could not be detected with the used apparatus (E). Addition of vanin inhibitor RR6 leads to the protection of CXP18.3-002, although some degradation is found leading to peaks corresponding to the amine and pantothenic acid (C). Addition of RR6 to CXP18.6-006 had no effect, since CXP18.6-006 was already stable in serum (F). Explanation of masses and compounds: CXP18.3-002, exact mass 322.189, found at mass 305 (-H₂O(18) +H⁺(1)). Amine from CXP18.3-002, exact mass 121.089, found at 163 (+ACN(41) +H⁺(1)). Pantothenic acid, exact mass 219.111, found at mass 220 (+H⁺(1)). RR6, exact mass 293.163, found at 294 (+H⁺(1)). CXP18.6-006, exact mass 322.189, found at mass 305 (-H₂O(18) +H⁺(1)). Amine from CXP18.6-006, exact mass 150.068, found at 151 (+H⁺(1)).

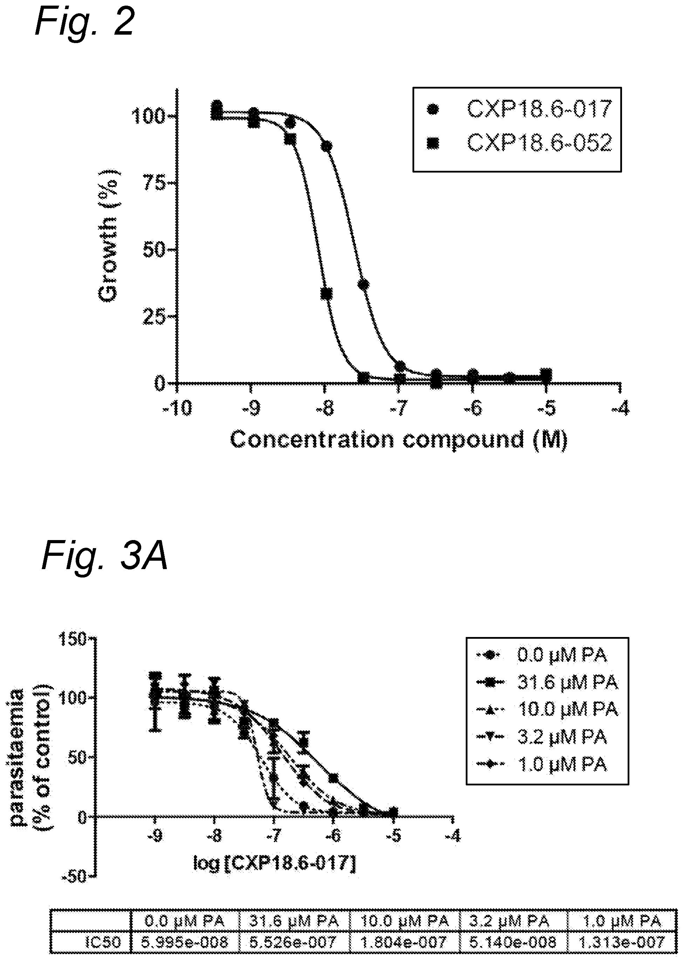

**Fig. 2****:** Inhibition of parasite growth by two inverted-amide pantothenamides. Effects on replication of *P. falciparum* asexual blood stage parasites. Asexual replication assays were performed in the presence of different doses of CXP18.6-017 and CXP18.6-052. The relative number of parasites was determined by a Sybrgreen DNA detection method. Data are expressed relative to the MAX (0.1% DMSO) and MIN (10 µM DHA) controls. Error bars indicate standard deviations from two independent observations.

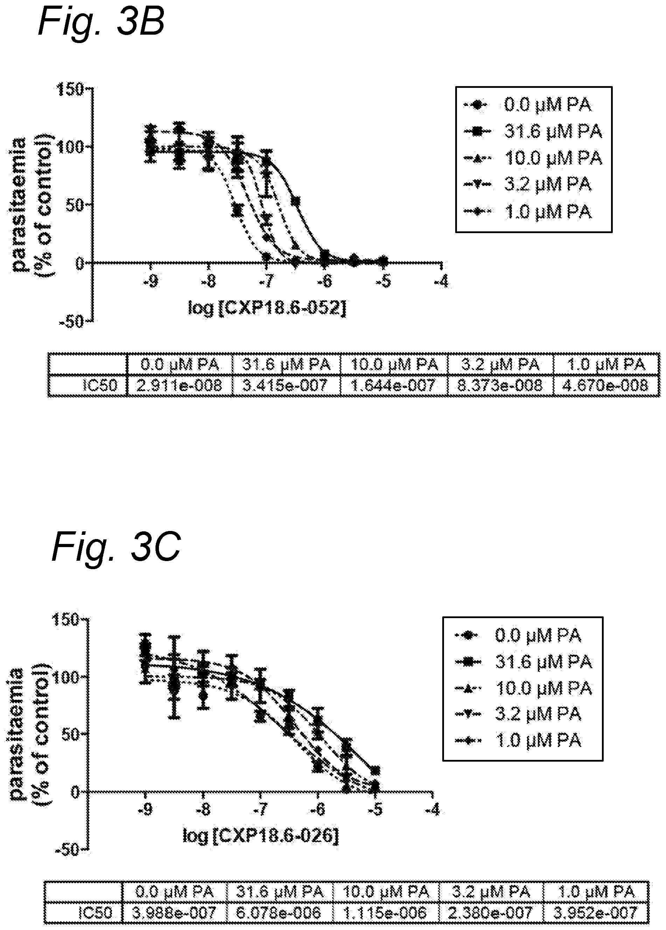

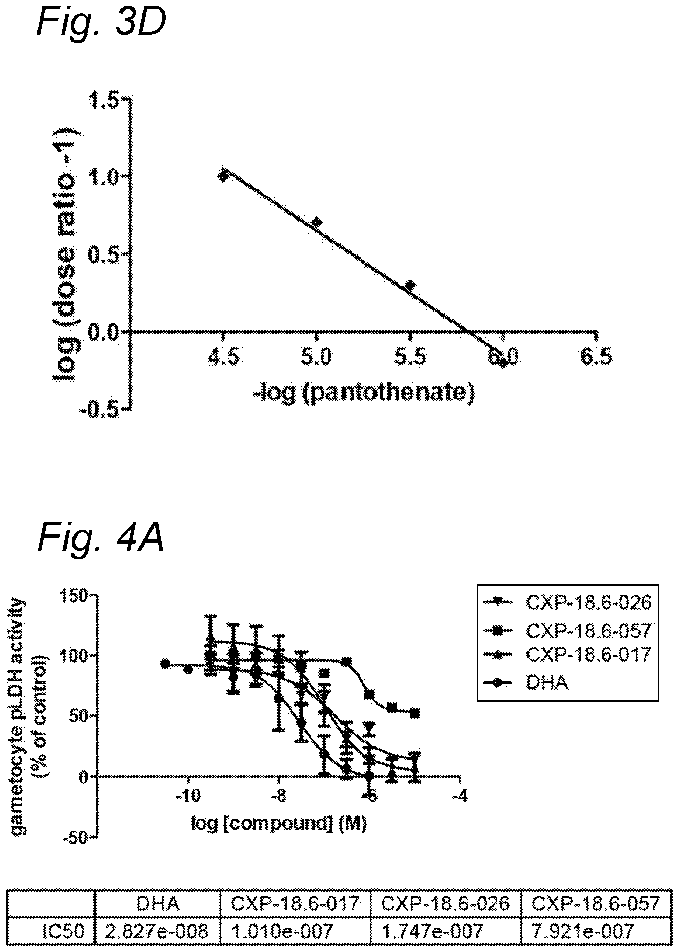

**Fig. 3****:** Competition between pantothenic acid and inverted-amide pantothenamide. Asexual replication assays were performed in combinations of pantothenic acid (PA) and CXP18.6-017 (A), CXP18.6-026 (B) and CXP18.6-052 (C). The relative number of parasites was determined by a Sybrgreen DNA detection method. Data are expressed relative to the MAX (0.1% DMSO) and MIN (10 µM DHA) controls. Error bars indicate standard deviations from two independent observations. D) Schild regression analysis of combinations of CXP18.6-052 and pantothenic acid.

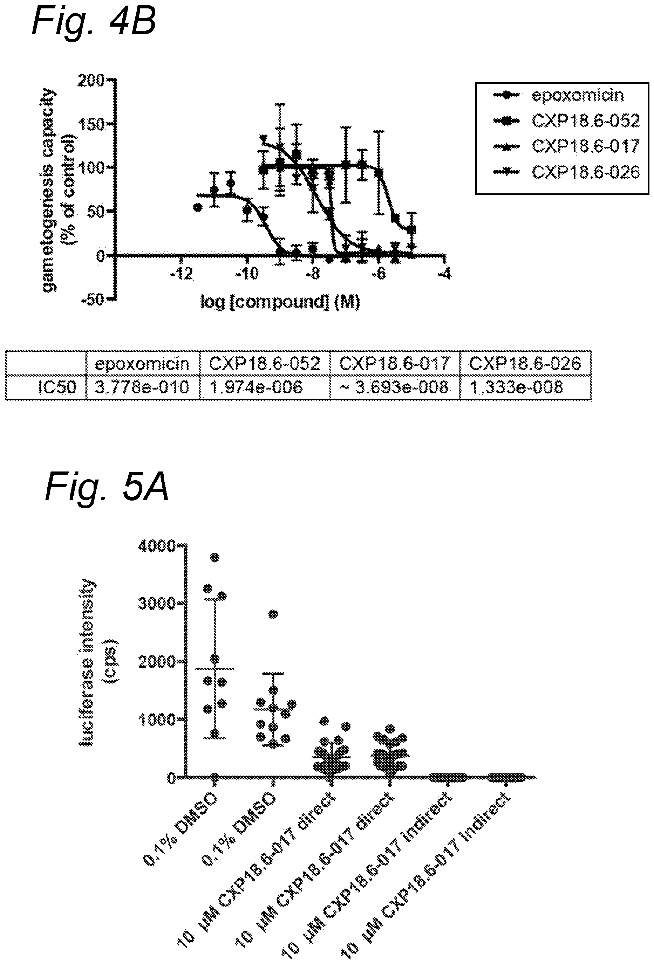

**Fig. 4****:** Malaria gametocytocidal activity of inverted-amide pantothenamides. Effects against *P. falciparum* gametocytes. A) Mature gametocytes were incubated with compound for three days and gametocyte viability was determined by measuring pLDH activity. B) Mature gametocytes were incubated with compound for three days, gametogenesis was induced and after 24 hours the relative number of female gametes was determined by detection of the Pfs25 antigen using an AlphaLisa method. Data are expressed relative to the MAX (0.1% DMSO) and MIN (10 µM DHA) controls. Error bars indicate standard deviations from two independent observations.

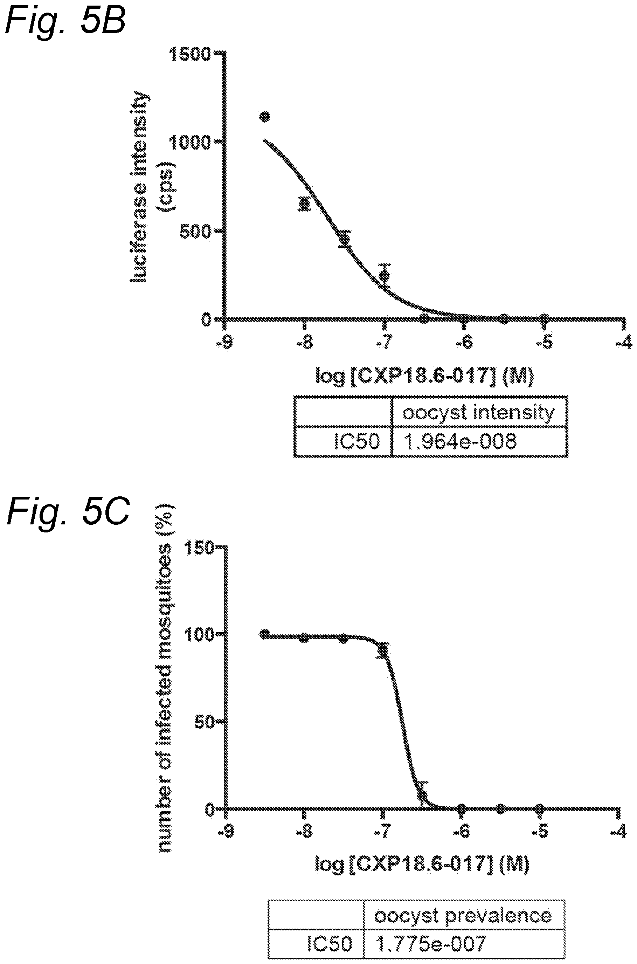

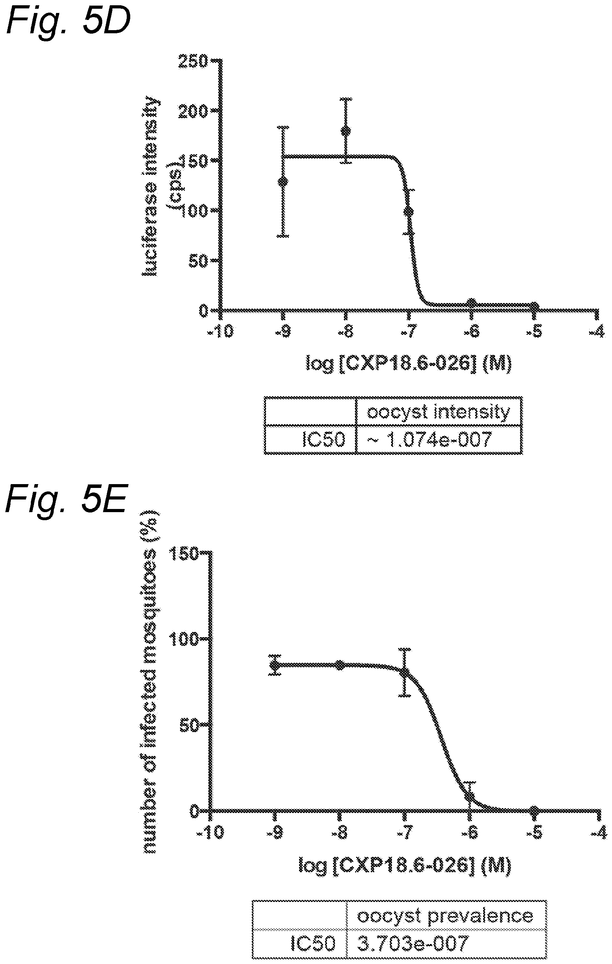

**Fig. 5****:** Inverted-amide pantothenamides block transmission of malaria parasites in Standard Membrane Feeding Assays. A) Luminescence activities in individual mosquitoes 8 days post feeding. Compound CXP18.6-017 was added to a parasite culture containing mature gametocytes 24 hours prior to mosquito infection (indirect mode) or directly to the bloodmeal at the moment of feeding (direct mode). B&D) Dose dependent reduction in parasite transmission. The figures show average luciferase activities determined in 2 x 24 mosquitoes from two independent cages. Error bars indicate standard error of the mean. C&E) Dose dependent effect on infection prevalence (% of parasite-infected mosquitoes). Mosquitoes were considered positive when luminescence signals were significantly greater than the background signal in uninfected mosquitoes (cut-off = *x̅* + 5σ). The figure shows the mean prevalence determined from two cages with 24 mosquitoes per cage. Error bars indicate standard deviations.

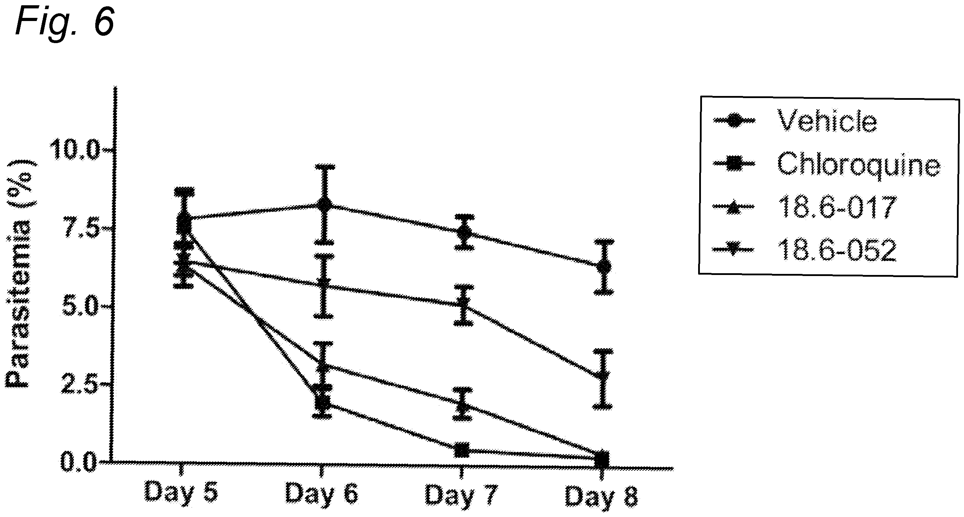

**Fig. 6****:** In vivo antimalarial activity of inverted-amide pantothenamides. Activity in an in vivo model for malaria. Female Wistar rats were inoculated with P. berghei malaria parasites and treated from day 5-8 with either vehicle, 6 mg/kg chloroquine, 100 mg/kg CXP18.6-017 or 100 mg/kg CXP18.6-017 by oral dosing three times a day. Parasitaemia was monitored by examination of Giemsa-stained blood smears.

### EXAMPLES

### Example 1: synthesis of pantothenamide analogues

### General information:

Unless noted otherwise, materials were purchased from commercial suppliers and used as received. All air and moisture sensitive reactions were carried out under an inert atmosphere of dry nitrogen. DCM was dried over Na₂SO₄ prior to use. Column chromatography was performed using Acros silica gel (0.035-0.070 mm, 6 nm). NMR spectra were recorded at 298 K on a Varian 400 (400 MHz) spectrometer in the solvent indicated. Chemical shifts are given in parts per million (ppm) with respect to tetramethylsilane (0.00 ppm), or CHD₂OD (3.31 ppm) as internal standard for ¹H NMR. Coupling constants are reported as *J* values in hertz (Hz).

### General procedure A:

To a solution of carboxylic acid **B** (0.5 mmol) in MeCN/H₂O (30:1, 4.3 mL) were added HOBt (0.6 mmol), NaHCO₃ (0.6 mmol), EDCI (0.6 mmol) and a solution of amine **A** (for the synthesis see ref 2, 0.6 mmol) in MeCN/H₂O (0.7 mL). The progress of the reaction was monitored using LC-MS and upon completion, the reaction was quenched by the addition of saturated aqueous NH₄Cl solution (15 mL) and the mixture was extracted twice using EtOAc (15 mL). The combined organic layers were dried over Na₂SO₄ and filtered before concentration under reduced pressure. The residue was purified by flash column chromatography (DCM/MeOH = 98:2 → 80:20) to afford the product. 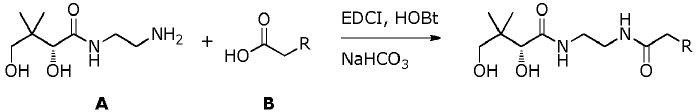
*CXP18.6-006:* According to general procedure A. Yield: 51%, pale yellow oil. ¹H NMR (400 MHz, CD₃OD): δ 7.30-7.15 (m, 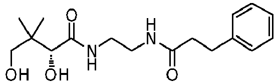 5H), 3.88 (s, 1H), 3.46 (d, *J* = 11.0 Hz, 1H), 3.36 (d, *J* = 11.0 Hz, 1H), 3.31-3.21 (m, 4H), 2.93-2.87 (m, 2H), 2.50-2.44 (m, 2H), 0.92 (s, 3H), 0.92 (s, 3H).
*CXP18.6-007:* According to general procedure A. Yield: 18%, colorless oil. ¹H NMR (400 MHz, CD₃OD): δ 7.33-7.22 (m, 5H), 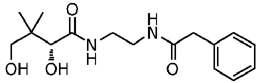 3.87 (s, 1H), 3.50 (s, 2H), 3.45 (d, *J* = 11.0 Hz, 1H), 3.38 (d, *J* = 11.0 Hz, 1H), 3.35-3.28 (m, 4H), 0.91 (s, 3H), 0.91 (s, 3H).
*CXP18.6-008:* According to general procedure A. Yield: 54%, colorless oil. ¹H NMR (400 MHz, CD₃OD): δ 7.27-7.12 (m, 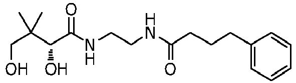 5H), 3.89 (s, 1H), 3.46 (d, *J* = 11.0 Hz, 1H), 3.46 (d, *J* = 11.0 Hz, 1H), 3.38-3.30 (m, 4H), 2.65-2.59 (m, 2H), 2.23-2.17 (m, 2H), 1.96-1.86 (m, 2H), 0.92 (s, 3H), 0.91 (s, 3H).
*CXP18.6-012:* According to general procedure A. Yield: 24%, white solid. ¹H NMR (400 MHz, CDCl₃): δ 7.23 (br s, 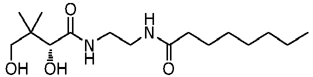 1H), 6.15 (br s, 1H), 4.01 (s, 1H), 3.84 (br s, 1H), 3.55-3.34 (m, 6H), 3.22 (br s, 1H), 2.20-2.14 (m, 2H), 1.70-1.55 (m, 2H), 1.35-1.20 (m, 8H), 1.04 (s, 3H), 0.96 (s, 3H), 0.88 (t, *J* = 6.9 Hz, 3H).
*CXP18.6-013:* According to general procedure A. Yield: 40%, white solid. ¹H NMR (400 MHz, CDCl₃): δ 7.28 (br s, 1H), 6.26 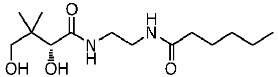 (br s, 1H), 4.10-3.97 (m, 2H), 3.55-3.30 (m, 7H), 2.21-2.13 (m, 2H) 1.65-1.55 (m, 2H), 1.38-1.22 (m, 4H), 1.03 (s, 3H), 0.95 (s, 3H), 0.88 (t, *J* = 7.0 Hz, 3H).
*CXP18.6-014:* According to general procedure A. Yield: 66%, white solid. ¹H NMR (400 MHz, CDCl₃): δ 7.27 (br 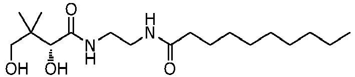 s, 1H), 6.23 (br s, 1H), 4.03-3.98 (m, 2H), 3.53-3.33 (m, 7H), 2.20-2.14 (m, 2H), 1.65-1.54 (m, 2H), 1.34-1.20 (m, 12H), 1.03 (s, 3H), 0.95 (s, 3H), 0.88 (t, *J* = 6.9 Hz, 3H).
*CXP18.6-017:* According to general procedure A. Yield: 35%, colorless oil. ¹H NMR (400 MHz, CD₃OD): δ 7.28-7.17 (m, 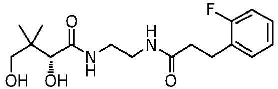 2H), 7.11-6.98 (m, 2H), 3.88 (s, 1H), 3.46 (d, *J* = 11.0 Hz, 1H), 3.39 (d, *J* = 11.0 Hz, 1H), 3.33-3.22 (m, 4H), 2.98-2.92 (m, 2H), 2.51-2.54 (m, 2H), 0.92 (s, 3H), 0.92 (s, 3H).
*CXP18.6-018:* According to general procedure A. Yield: 35%, colorless oil. ¹H NMR (400 MHz, CD₃OD): δ 7.27 (td, *J* = 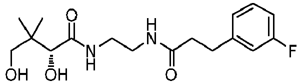 7.9, 6.1 Hz, 1H), 7.04-7.00 (m, 1H), 6.98-6.88 (m, 2H), 3.88 (s, 1H), 3.46 (d, *J* = 11.0 Hz, 1H), 3.39 (d, *J* = 11.0 Hz, 1H), 3.33-3.22 (m, 4H), 2.95-2.88 (m, 2H), 2.51-2.44 (m, 2H), 0.92 (s, 3H), 0.92 (s, 3H).
*CXP18.6-019:* According to general procedure A. Yield: 44%, colorless oil. ¹H NMR (400 MHz, CD₃OD): δ 7.27-7.13 (m, 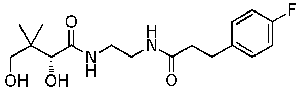 2H), 7.05-6.91 (m, 2H), 3.88 (s, 1H), 3.46 (d, *J* = 11.0 Hz, 1H), 3.39 (d, *J* = 11.0 Hz, 1H), 3.33-3.21 (m, 4H), 2.92-2.86 (m, 2H), 2.48-2.42 (m, 2H), 0.92 (s, 3H), 0.92 (s, 3H).
*CXP18.6-022:* According to general procedure A. Yield: 52%, colorless oil. ¹H NMR (400 MHz, CD₃OD): δ 7.14-7.09 (m, 2H), 6.85-6.79 (m, 2H), 3.88 (s, 1H), 3.75 (s, 3H), 3.46 (d, *J* = 11.0 Hz, 1H), 3.39 (d, *J=* 11.0 Hz, 1H), 3.33-3.21 (m, 4H), 2.88-2.78 (m, 2H), 2.47-2.38 (m, 2H), 0.92 (s, 3H), 0.92 (s, 3H). 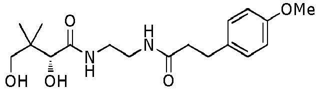
*CXP18.6-026:* According to general procedure A. Yield: 56%, colorless oil. ¹H NMR (400 MHz, CD₃OD): δ 7.08 (app s, 4H), 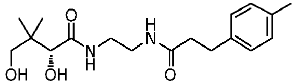 3.88 (s, 1H), 3.46 (d, *J* = 11.0 Hz, 1H), 3.39 (d, *J* = 11.0 Hz, 1H), 3.33-3.21 (m, 4H), 2.88-2.82 (m, 2H), 2.47-2.40 (m, 2H), 2.28 (s, 3H), 0.92 (s, 3H), 0.92 (s, 3H).
*CXP18.6-027:* According to general procedure A. Yield: 55%, colorless oil. ¹H NMR (400 MHz, CD₃OD): δ 7.38-7.34 (m, 1H), 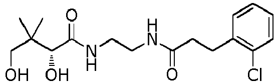 7.32-7.27 (m, 1H), 7.25-7.16 (m, 2H), 3.89 (s, 1H), 3.46 (d, *J* = 11.0 Hz, 1H), 3.39 (d, *J* = 11.0 Hz, 1H), 3.35-3.23 (m, 4H), 3.07-3.01 (m, 2H), 2.53-2.46 (m, 2H), 0.92 (s, 3H), 0.92 (s, 3H).
*CXP18.6-028:* According to general procedure A. Yield: 54%, colorless oil. ¹H NMR (400 MHz, CD₃OD): δ 7.29-7.23 (m, 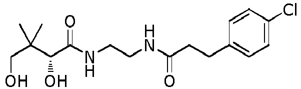 2H), 7.23-7.16 (m, 2H), 3.88 (s, 1H), 3.46 (d, *J* = 11.0 Hz, 1H), 3.39 (d, *J* = 11.0 Hz, 1H), 3.33-3.21 (m, 4H), 2.95-2.85 (m, 2H), 2.50-2.41 (m, 2H), 0.92 (s, 3H), 0.92 (s, 3H).
*CXP18.6-038:* According to general procedure A. Yield: 71%, colorless oil. ¹H NMR (400 MHz, CD₃OD): δ 7.65 (d, *J* = 8.0 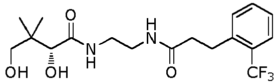 Hz, 1H), 7.55 (t, *J* = 7.6 Hz, 1H), 7.44 (d, *J* = 7.8 Hz, 1H), 7.37 (t, *J* = 7.6 Hz, 1H), 3.89 (s, 1H), 3.46 (d, *J* = 11.0 Hz, 1H), 3.39 (d, *J* = 11.0 Hz, 1H), 3.37-3.26 (m, 4H), 3.14-3.05 (m, 2H), 2.52-2.45 (m, 2H), 0.92 (s, 3H), 0.92 (s, 3H).
*CXP18.6-042:* According to general procedure A. Yield: 73%, white solid. ¹H NMR (400 MHz, CD₃OD): δ 7.57 (d, *J* 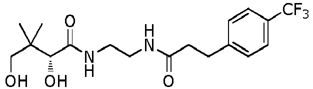 = 8.0 Hz, 1H), 7.41 (d, *J* = 8.0 Hz, 1H), 3.88 (s, 1H), 3.46 (d, *J* = 11.0 Hz, 1H), 3.39 (d, *J* = 11.0 Hz, 1H), 3.33-3.22 (m, 4H), 3.04-2.94 (m, 2H), 2.56-2.45 (m, 2H), 0.92 (s, 3H), 0.91 (s, 3H).
*CXP18.6-043:* According to general procedure A. Yield: 82%, colorless oil. ¹H NMR (400 MHz, CD₃OD): δ 7.33-7.23 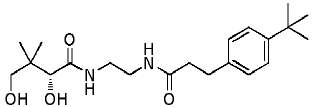 (m, 2H), 7.16-7.09 (m, 2H), 3.89 (s, 1H), 3.46 (d, *J* = 11.0 Hz, 1H), 3.39 (d, *J* = 11.0 Hz, 1H), 3.33-3.20 (m, 4H), 2.90-2.83 (m, 2H), 2.48-2.42 (m, 2H), 1.29 (s, 9H), 0.92 (s, 3H), 0.92 (s, 3H).
*CXP18.6-046:* According to general procedure A. Yield: 21%, colorless oil. ¹H NMR (400 MHz, CD₃OD): δ 7.31 (dd, *J* = 4.9, 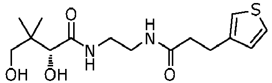 3.0 Hz, 1H), 7.07-07.03 (m, 1H), 6.97 (dd, *J* = 5.0, 1.3 Hz, 1H), 3.89 (s, 1H), 3.46 (d, *J* = 11.0 Hz, 1H), 3.39 (d, *J* = 11.0 Hz, 1H), 3.33-3.22 (m, 4H), 2.97-2.89 (m, 2H), 2.51-2.44 (m, 2H), 0.93 (s, 3H), 0.92 (s, 3H).
*CXP18.6-047:* According to general procedure A. Yield: 25%, colorless oil. ¹H NMR (400 MHz, CD₃OD): δ 7.18 (dd, *J* = 5.1, 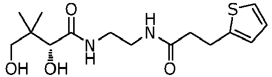 1.2 Hz, 1H), 6.90 (dd, *J* = 5.1, 3.4 Hz, 1H), 6.86-6.81 (m, 1H), 3.87 (s, 1H), 3.46 (d, *J* = 11.0 Hz, 1H), 3.39 (d, *J* = 11.0 Hz, 1H), 3.35-3.23 (m, 4H), 3.17-3.09 (m, 2H), 2.56-2.49 (m, 2H), 0.92 (s, 3H), 0.92 (s, 3H).
*CXP18.6-048:* According to general procedure A. Yield: 24%, white solid. ¹H NMR (400 MHz, CD₃OD): δ 7.58-7.50 (m, 3H), 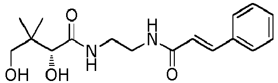 7.42-7.34 (m, 3H), 6.59 (d, *J* = 15.8 Hz, 1H), 3.91 (s, 1H), 3.50-3.34 (m, 6H), 0.93 (s, 3H), 0.92 (s, 3H).
*CXP18.6-057:* According to general procedure A. Yield: 48%, off-white solid. ¹H NMR (400 MHz, CD₃OD): δ 7.35 (dd, *J* = 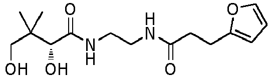 1.9, 0.8 Hz, 1H), 6.28 (dd, *J* = 3.2, 1.9 Hz, 1H), 3.89 (s, 1H), 3.46 (d, *J* = 11.0 Hz, 1H), 3.39 (d, *J* = 11.0 Hz, 1H), 3.36-3.25 (m, 4H), 2.96-2.86 (m, 2H), 2.55-2.45 (m, 2H), 0.93 (s, 3H), 0.92 (s, 3H).
*CXP18.6-064:* According to general procedure A. Yield: 92%, colorless oil. ¹H NMR (400 MHz, CD₃OD): δ 7.10 (app t, *J* = 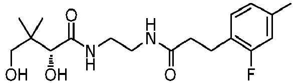 7.9 Hz, 1H), 6.92-6.83 (m, 2H), 3.88 (s, 1H), 3.46 (d, *J* = 11.0 Hz, 1H), 3.39 (d, *J* = 11.0 Hz, 1H), 3.33-3.22 (m, 4H), 2.94-2.85 (m, 2H), 2.48-2.40 (m, 2H), 2.30 (s, 3H), 0.92 (s, 3H), 0.91 (s, 3H).
*CXP18.6-069:* According to general procedure A. Yield: 43%, colorless oil. ¹H NMR (400 MHz, CD₃OD): δ 7.30-7.23 (m, 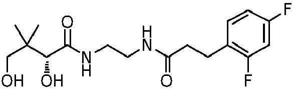 1H), 6.93-6.83 (m, 2H), 3.88 (s, 1H), 3.46 (d, *J* = 11.0 Hz, 1H), 3.39 (d, *J* = 11.0 Hz, 1H), 3.33-3.22 (m, 4H), 2.92 (app t, *J* = 7.7 Hz, 2H), 2.48-2.42 (m, 2H), 0.92 (s, 3H), 0.92 (s, 3H).
*CXP14.18-005:* According to general procedure A. Yield: 25%, colorless oil. ¹H NMR (400 MHz, CDCl₃): δ 7.30 (br s, 1H), 6.32 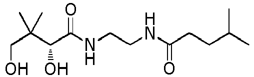 (br s, 1H), 4.02 (s, 1H), 3.51 (s, 2H), 3.49-3.34 (m, 4H), 2.22-2.15 (m, 2H), 1.62-1.46 (m, 3H), 1.03 (s, 3H), 0.96 (s, 3H), 0.91 (s, 3H), 0.89 (s, 3H).
*CXP14.18-007:* According to general procedure A. Yield: 29%, colorless oil. ¹H NMR (400 MHz, CD₃OD): δ 5.83 (ddt, *J* = 16.9, 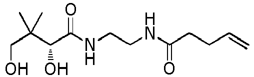 10.2, 6.3 Hz, 1H), 5.06 (ddd, *J* = 16.9, 3.4, 1.6 Hz, 1H), 5.01-4.95 (m, 1H), 3.89 (s, 1H), 3.46 (d, *J* = 11.0 Hz, 1H), 3.39 (d, *J* = 11.0 Hz, 1H), 3.38-3.25 (m, 4H), 2.38-2.30 (m, 2H), 2.30-2.23 (m, 2H), 0.93 (s, 3H), 0.92 (s, 3H).
*CXP14.18-008:* According to general procedure A. Yield: 41%, colorless oil. ¹H NMR (400 MHz, CD₃OD): δ 5.81 (ddt, *J =* 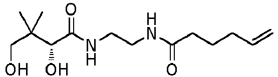 17.0, 10.2, 6.7 Hz, 1H), 5.03 (ddt, *J* = 17.0, 2.1, 1.6 Hz, 1H), 4.97 (ddt, *J* = 10.2, 2.1, 1.2 Hz, 1H), 3.89 (s, 1H), 3.46 (d, *J* = 11.0 Hz, 1H), 3.39 (d, *J* = 11.0 Hz, 1H), 3.39-3.26 (m, 4H), 2.24-2.14 (m, 2H), 2.13-2.01 (m, 2H), 1.76-1.59 (m, 2H), 0.93 (s, 3H), 0.92 (s, 3H).
*CXP14.18-010:* According to general procedure A. Yield: 66%, colorless oil. ¹H NMR (400 MHz, CD₃OD): δ 5.81 (ddt, 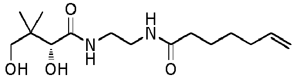 *J* = 17.0, 10.2, 6.7 Hz, 1H), 5.00 (ddt, *J* = 17.0, 2.1, 1.6 Hz, 1H), 4.93 (ddt, *J* = 10.2, 2.1, 1.2 Hz, 1H), 3.89 (s, 1H), 3.46 (d, *J* = 11.0 Hz, 1H), 3.39 (d, *J* = 11.0 Hz, 1H), 3.39-3.26 (m, 4H), 2.22-2.15 (m, 2H), 2.12-2.03 (m, 2H), 1.67-1.56 (m, 2H), 1.46-1.35 (m, 2H), 0.93 (s, 3H), 0.92 (s, 3H). 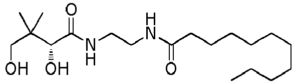
*CXP14.18-012:* According to general procedure A. Yield: 88%, white solid. ¹H NMR (400 MHz, CDCl₃): δ 7.28 (br s, 1H), 6.26 (br s, 1H), 4.01 (s, 1H), 3.51 (s, 2H), 3.50-3.31 (m, 4H), 2.20-2.14 (m, 2H), 1.65-1.55 (m, 2H), 1.37-1.18 (m, 16H), 1.30 (s, 3H), 0.95 (s, 3H), 0.88 (t, *J* = 6.9 Hz, 3H).
*CXP14.18-014:* According to general procedure A. Yield: 25%, colorless oil. ¹H NMR (400 MHz, CD₃OD): δ 5.77 (ddd, *J* = 17.3, 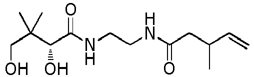 10.4, 7.2, 1H), 5.02 (dt, *J* = 17.3, 1.5 Hz, 1H), 4.99-4.91 (m, 1H), 3.89 (s, 1H), 3.47 (d, *J* = 10.9 Hz, 1H), 3.40 (d, *J* = 10.9 Hz, 1H), 3.38-3.26 (m, 4H), 2.68-2.56 (m, 1H), 2.21 (dd, *J* = 13.6, 7.4 Hz, 1H), 2.12 (dd, *J* = 13.6, 7.6 Hz, 1H), 1.03 (d, *J* = 6.8 Hz, 3H), 0.93 (s, 3H), 0.92 (s, 3H).
*CXP14.18-016:* According to general procedure A. Yield: 36%, colorless oil. ¹H NMR (400 MHz, CD₃OD): δ 3.90 (s, 1H), 3.47 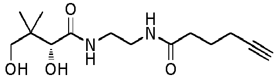 (d, *J* = 11.0 Hz, 1H), 3.40 (d, *J* = 11.0 Hz, 1H), 3.38-3.26 (m, 4H), 2.34-2.28 (m, 2H), 2.26-2.19 (m, 3H), 1.85-1.75 (m, 2H), 0.93 (s, 3H), 0.93 (s, 3H).

### General procedure B:

To a suspension of carboxylic acid B (0.72 mmol) in MeCN/H₂O (19:1, 4 mL) was added EDCI (0.79 mmol). After stirring for 10 min an almost clear solution was obtained. Amine A (for the synthesis see ref 2, 0.79 mmol) was added, followed by DIPEA (0.79 mmol). The progress of the reaction was monitored using LC-MS and upon completion, silica gel was added. The mixture was then concentrated under reduced pressure and purified by flash column chromatography (MeCN/MeOH = 4:1 → 72:1) to afford the product. 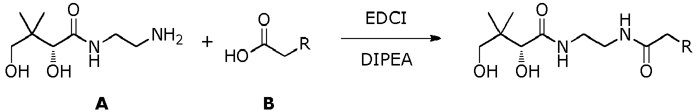
*CXP18.6-052:* According to general procedure B. Yield: 30%, colorless oil. ¹H NMR (400 MHz, CD₃OD): δ 8.42 (d, *J* = 1.8 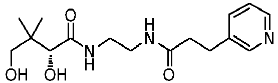 Hz, 1H), 8.37 (dd, *J* = 4.9, 1.6 Hz, 1H), 7.72 (ddd, *J* = 7.9, 2.2, 1.7 Hz, 1H), 7.37 (ddd, *J* = 7.8, 4.9, 0.8 Hz, 1H), 3.88 (s, 1H), 3.46 (d, *J* = 11.0 Hz, 1H), 3.39 (d, *J* = 11.0 Hz, 1H), 3.30-3.20 (m, 4H), 2.96 (t, *J* = 7.5 Hz, 2H), 2.51 (t, *J* = 7.5 Hz, 2H), 0.92 (s, 3H), 0.91 (s, 3H).
*CXP18.6-055:* According to general procedure B. Yield: 38%, colorless oil. ¹H NMR (400 MHz, CD₃OD): δ 8.45 (ddd, *J* = 5.0, 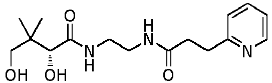 1.8, 0.9 Hz, 1H), 7.75 (td, *J* = 7.7, 1.8 Hz, 1H), 7.32 (dt, *J* = 7.9, 1.0 Hz, 1H7.25 (ddd, *J* = 7.5, 5.0, 1.2 Hz, 1H), 3.89 (s, 1H), 3.46 (d, *J* = 11.0 Hz, 1H), 3.39 (d, *J* = 11.0 Hz, 1H), 3.30-3.20 (m, 4H), 3.10-3.04 (m, 2H), 2.59 (dd, *J* = 8.3, 7.1 Hz, 2H), 0.93 (s, 3H), 0.92 (s, 3H).
*CXP18.6-056:* According to general procedure B. Yield: 33%, white solid. ¹H NMR (400 MHz, CD₃OD): δ 8.42 (dd, *J* = 4.5, 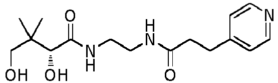 1.6 Hz, 1H), 7.31 (dd, *J* = 4.6, 1.6 Hz, 1H), 3.88 (s, 1H), 3.46 (d, *J* = 11.0 Hz, 1H), 3.39 (d, *J* = 11.0 Hz, 1H), 3.35-3.22 (m, 4H), 2.97 (t, *J* = 7.6 Hz, 2H), 2.53 (t, *J* = 7.6 Hz, 2H), 0.92 (s, 3H), 0.92 (s, 3H).

### General procedure C:

To a solution of carboxylic acid **B** (2.2 mmol) in DCM (6 mL) were added EDCI (2.2 mmol) and a solution of amine **C** (prepared analogously to amine **A**, 1.0 mmol) in DCM (4 mL). The progress of the reaction was monitored using LC-MS and upon completion, the reaction was quenched by the addition of saturated aqueous NH₄Cl solution (20 mL) and the mixture was extracted twice using DCM (15 mL). The combined organic layers were dried over Na₂SO₄ and filtered before concentration under reduced pressure. The residue was purified by flash column chromatography (DCM/MeOH = 98:2 → 80:20) to afford the product. 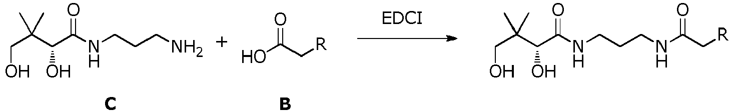
*CXP18.6-009:* According to general procedure C. Yield: 30%, colorless oil. ¹H NMR (400 MHz, CD₃OD): δ 7.34-7.20 (m, 5H), 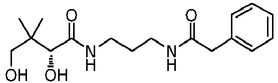 3.89 (s, 1H), 3.50 (s, 2H), 3.47 (d, *J* = 10.9 Hz, 1H), 3.38 (d, *J* = 10.9 Hz, 1H), 3.28-3.14 (m, 4H), 1.67 (p, *J* = 6.7 Hz, 2H), 0.93 (s, 6H).
*CXP18.6-010:* According to general procedure C. Yield: 58%, colorless oil. ¹H NMR (400 MHz, CD₃OD): δ 7.29-7.14 (m, 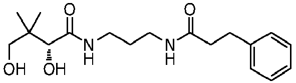 5H), 3.89 (s, 1H), 3.48 (d, *J* = 10.9 Hz, 1H), 3.39 (d, *J* = 10.9 Hz, 1H), 3.21-3.01 (m, 4H), 2.91 (t, *J* = 7.6 Hz, 2H), 2.52-2.45 (m, 2H), 1.58 (p, *J* = 6.7 Hz, 2H), 0.93 (s, 6H).

### Synthesis of CXP14.18-037:

Amine A (1.50 g, 7.88 mmol) was dissolved in acetone (25 mL) and cooled to 4 °C. 2-Methoxyprop-1-ene (2.26 mL, 23.6 mmol) and TsOH (1.65 g, 8.67 mmol) were added and after 15 min the cooling bath was removed. The reaction was stirred for 18 h and then quenched by the addition of Et₃N (1.60 g, 15.7 mmol), followed by 12.5% aqueous ammonia (15 mL). The mixture was extracted twice with DCM (15 mL) and the combined organic layers were concentrated under reduced pressure. The residue was purified by flash column chromatography (EtOAc/MeOH/25% aq. NH₄OH = 90:10:1 → 50:50:1) to afford 1.07 g amine **D** as a colorless oil. Yield: 30%. ¹H NMR (400 MHz, CD₃OD): δ 4.15 (s, 1H), 3.75 (ABd, *J* = 11.7 Hz, 1H), 3.24-3.30 (m, 3H), 2.73 (m, 2H), 1.46 (s, 3H), 1.45 (s, 3H), 1.00 (s, 3H), 0.99 (s, 3H).

To a solution of 2-(propylthio)acetic acid (0.15 g, 0.81 mmol) in a mixture of MeCN (4.4 mL) and H₂O (0.24 mL) were added HOBt (0.11 g, 0.71 mmol), DIPEA (0.14 mL, 0.81 mmol), EDC (0.14 g, 0.72 mmol) and amine **D** (0.15 g, 0.65 mmol). The reaction was stirred at room temperature for 2 h, before the addition of EtOAc (10 mL). The mixture washed with saturated aqueous NH₄Cl (10 mL), and saturated aqueous NaHCO₃ (10 mL), dried over Na₂SO₄ and filtered before concentration under reduced pressure. The residue was purified by flash column chromatography (EtOAc/MeOH = 95:5 → 90:10) to afford amide **E** (147 mg, 65%).

To a solution of amide **E** (0.15 mg, 0.42 mmol) in MeCN (2.1 mL) was added an aqueous 0.2 M solution of HCl (2.12 mL, 0.42 mmol). The mixture was stirred at room temperature for 2 h. The mixture was added dropwise to a mixture of saturated aqueous NaHCO₃ (2 mL) and EtOAc (10 mL). The layers were separated and the aqueous phase was saturated with NaCl and then extracted with EtOAc (10 mL). The combined organic layers were dried over Na₂SO₄ and filtered before concentration under reduced pressure to afford CXP14.18-037 (113 mg, 85%) as a pale yellow oil. ¹H NMR (400 MHz, CDCl₃): δ 7.30 (br s, 1H), 6.30 (br s, 1H), 4.13 (d, *J* = 5.0 Hz, 1H), 4.01 (d, *J* = 5.0 Hz, 1H), 3.55-3.33 (m, 7H), 2.22-2.15 (m, 2H), 1.65-1.53 (m, 2H), 1.39-1.25 (m, 2H), 1.03 (s, 3H), 0.95 (s, 3H), 0.91 (t, *J* = 7.3 Hz, 3H). 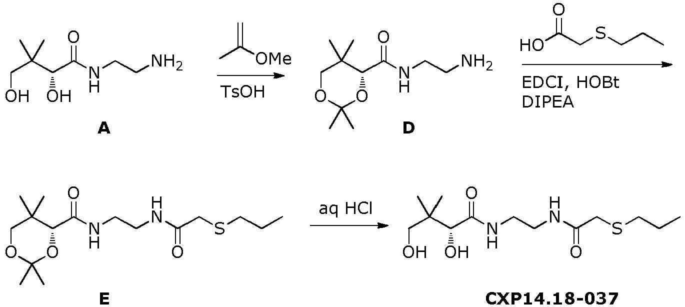
*CXP14.18-019:* According to the sequence used for CXP14.18-037. Yield (from **D**): 28%, white solid. ¹H NMR (400 MHz, CD₃OD): δ 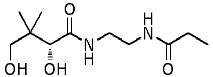 3.89 (s, 1H), 3.46 (d, *J* = 10.9 Hz, 1H), 3.39 (d, *J* = 10.9 Hz, 1H), 3.40-3.28 (m, 4H), 2.20 (q, *J* = 7.6 Hz, 2H), 1.12 (t, *J* = 7.6 Hz, 3H), 0.93 (s, 3H), 0.92 (s, 3H).
*CXP14.18-020:* According to the sequence used for CXP14.18-037. Yield (from **D**): 44%, colorless oil. ¹H NMR (400 MHz, CD₃OD): δ 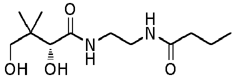 3.89 (s, 1H), 3.47 (d, *J* = 10.9 Hz, 1H), 3.39 (d, *J* = 10.9 Hz, 1H), 3.40-3.28 (m, 4H), 2.20-2.12 (m, 2H), 1.70-1.56 (m, 2H), 0.94 (t, *J* = 7.4 Hz, 3H), 0.93 (s, 3H), 0.92 (s, 3H).
*CXP14.18-028:* According to the sequence used for CXP14.18-037. Yield (from **D**): 55%, colorless oil. ¹H NMR (400 MHz, 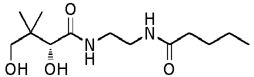 CD₃OD): δ 3.89 (s, 1H), 3.47 (d, *J* = 10.9 Hz, 1H), 3.39 (d, *J* = 10.9 Hz, 1H), 3.40-3.28 (m, 4H), 2.21-2.15 (m, 2H), 1.65-1.53 (m, 2H), 1.44-1.29 (m, 2H), 0.94-0.91 (m, 9H).
*CXP14.18-034:* According to the sequence used for CXP14.18-037. Yield (from **D**): 37%, pale yellow oil. ¹H NMR (400 MHz, 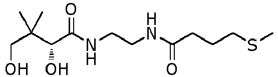 CDCl₃): δ 7.25 (br s, 1H), 6.32 (br s, 1H), 4.02 (d, *J* = 5.1 Hz, 1H), 3.97 (d, *J* = 5.1 Hz, 1H), 3.55-3.27 (m, 7H), 2.53 (t, *J* = 7.0 Hz, 2H), 2.31 (t, *J* = 7.4 Hz, 2H), 2.09 (s, 3H), 1.99-1.87 (m, 2H), 1.03 (s, 3H), 0.95 (s, 3H).
*CXP14.18-035:* According to the sequence used for CXP14.18-037. Yield (from **D**): 34%, colorless oil. ¹H NMR (400 MHz, 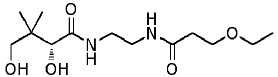 CDCl₃): δ 7.25 (br s, 1H), 6.65 (br s, 1H), 3.99 (d, *J* = 5.4 Hz, 1H), 3.95 (d, *J* = 5.5 Hz, 1H), 3.74-3.61 (m, 2H), 3.57-3.35 (m, 8H), 2.46 (t, *J* = 5.5 Hz, 2H), 1.22 (t, *J* = 7.0 Hz, 3H), 1.04 (s, 3H), 0.95 (s, 3H).
*CXP14.18-038:* According to the sequence used for CXP14.18-037. Yield (from **D**): 34%, colorless oil. ¹H NMR (400 MHz, 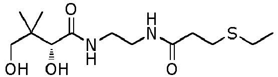 CDCl₃): δ 7.28 (br s, 1H), 6.44 (br s, 1H), 4.03-3.93 (m, 1H), 3.55-3.33 (m, 7), 2.90-2.70 (m, 2H), 2.55 (q, *J* = 7.8 Hz, 2H), 2.53-2.38 (m, 2H), 1.26 (t, *J* = 7.8 Hz, 3H), 1.04 (s, 3H), 0.95 (s, 3H).

### Synthesis of MMV689258 (Panto-26)

To a stirred solution of ((S)-2-Hydroxy-1-methyl-ethyl)-carbamic acid tert-butyl ester **1** (2.5 g, 14.26 mmol) in dry THF (28 ml) were added phthalimide **2** (2.3g, 15.69 mmol) and PPh3 (4.11g, 15.69 mmol). DEAD (2.73 g, 15.69 mmol) was then added dropwise to the stirred solution at room temperature and maintained for 16h. The reaction mixture was then concentrated under reduced pressure and the residue was purified by column chromatography (70:30 -50:50 hexanes-EtOAc) to afford compound **3** (4 g, 92%) as white solid.

To a stirred solution of compound **3** (2.0 g, 6.57 mmol) in dioxane (20 ml) was added 4M dioxane-HCl (20 ml) at 0°C. It was stirred at RT for 6h. It was concentrated under reduced pressure to afford compound **4** (1.5 g, 95%) as white solid.

To a stirred solution of compound **5** (180 mg, 1.071 mmol) in THF (10 ml) were added Et3N (0.747 ml, 5.357 mmol), HATU (611 mg, 1.607 mmol) and compound **4** (515 mg, 2.143 mmol). It was stirred at room temp for 16h. TLC (30% EtOAc-Hexane) showed completion of the reaction. It was diluted with water (20 ml) and extracted with EtOAc (50 ml), washed with brine (10 ml), dried over Na2SO4 and concentrated under reduced pressure. It was purified by combiflash (30% EtOAc-Hexane) to afford compound **6** (330 mg, 87%) as off white solid.

To a stirred solution of compound **6** (330 mg, 0.931 mmol) in EtOH (20 ml) was added hydrazine hydrate (744.96 mg, 14.899 mmol). It was heated at 50°C for 2h. It was cooled to room temp, filtered and concentrated under reduced pressure. The resulting residue was suspended in Et2O (20 ml) and filtered, washing thoroughly with Et2O (20 ml). The combined filtrates were concentrated under reduced pressure to afford compound **7** (150 mg, 72 %) as gum.

To a stirred solution of compound **8** (75 mg, 0.318 mmol) in THF (20 ml) were added Et3N (0.221ml, 1.589 mmol), HATU (181.25 mg, 0.477 mmol) and compound 7 (142.37 mg, 0.636 mmol). It was stirred at room temp for 16h. It was diluted with water (20 ml) and extracted with EtOAc (50 ml), washed with satd NaHCO₃ (20 ml), brine (10 ml), dried over Na2SO4 and concentrated under reduced pressure. It was purified by column chromatography (3% MeOH-DCM) to afford compound **9** (110 mg, 78%) as gum.

Compound **9** (90 mg, 0.203 mmol) was taken in MeOH (10 ml) and was degassed with argon for 10 minutes. Pd/C (50 mg, 10% moist) was added and the mixture was subjected to hydrogenation in a Parr vessel at 50psi for 16h. It was filtered through celite, concentrated under reduced pressure and purified by preparative TLC (4% MeOH-DCM) to afford MMV689258 (40 mg, 55 %) as colourless gum. ¹H NMR (400 MHz, DMSO-*d₆*) δ 0.79 (d, 6 H), 0.95 (d, 3H), 2.32 (t, 2H), 2.81 (t, 2H), 2.99-3.02 (m, 1H), 3.12-3.18 (m, 2H), 3.27-3.32 (m, 1H), 3.72 (d, 1H), 3.83-3.86 (m, 1H), 4.46 (t, 1H, -OH), 5.38 (d, 1H, -OH), 7.08-7.14 (m, 2H), 7.21-7.28 (m, 2H), 7.68-7.72 (m, 2H).

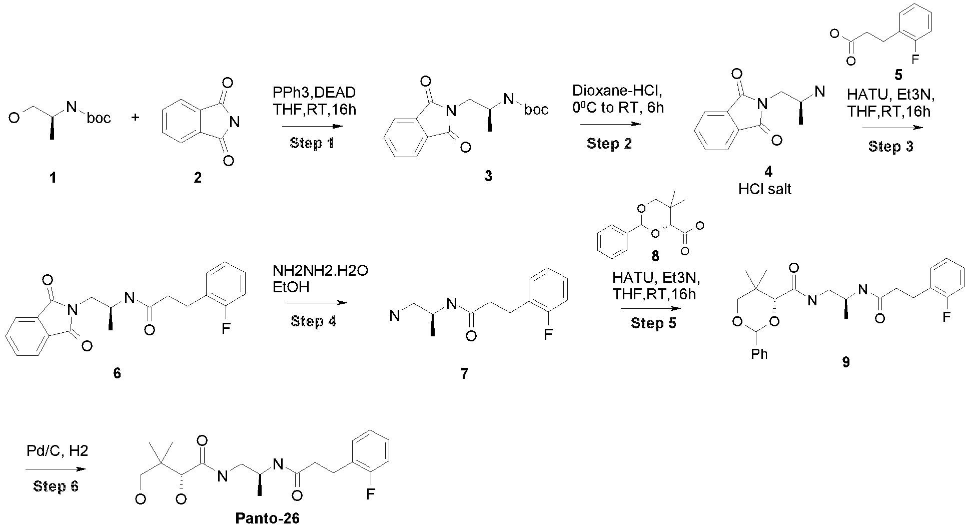

### Synthesis of MMV689260 (Panto-28)

To a stirred solution of (R)-tert-butyl 1-hydroxypropan-2-ylcarbamate **1** (2.5 g, 14.26 mmol) in dry THF (28 ml) were added phthalimide **2** (2.3g, 15.69 mmol) and PPh3 (4.11g, 15.69 mmol). DEAD (2.73 g, 15.69 mmol) was then added dropwise to the stirred solution at room temperature and maintained for 16h. The reaction mixture was then concentrated under reduced pressure and the residue was purified by column chromatography (70:30 -50:50 hexanes-EtOAc) to afford compound 3 (4.2 g, 97%) as white solid.

To a stirred solution of compound **3** (1.5 g, 4.93 mmol) in dioxane (15 ml) was added 4M dioxane-HCl (15 ml) at 0°C. It was stirred at room temperature for 6h. It was concentrated under reduced pressure to afford compound **4** (1.1 g, 93%) as white solid.

To a stirred solution of compound **5** (350 mg, 2.08 mmol) in THF (10 ml) were added Et3N (1.452 ml, 10.42 mmol), HATU (1188 mg, 3.12 mmol) and compound 4 (752 mg, 3.12 mmol). It was stirred at room temperature for 16h. TLC (30% EtOAc-Hexane) showed completion of the reaction. It was diluted with water (20 ml) and extracted with EtOAc (50 ml), washed with brine (10 ml), dried over Na2SO4 and concentrated under reduced pressure. It was purified by combiflash (30% EtOAc-Hexane) to afford compound **6** (730 mg, 99%) as off white solid.

To a stirred solution of compound **6** (730 mg, 2.06 mmol) in MeOH (20 ml) was added hydrazine hydrate (1.65 g, 32.95 mmol). It was heated at 50°C for 2h. It was cooled to room temperature, filtered and concentrated under reduced pressure. The resulting residue was suspended in Et2O (20 ml) and filtered, washing the solid thoroughly with Et2O (20 ml). The combined filtrates were concentrated under reduced pressure to afford compound 7 (460 mg, 99%) as gum.

To a stirred solution of compound **8** (130 mg, 0.55 mmol) in THF (10 ml) were added Et3N (0.384ml, 2.75 mmol), HATU (314 mg, 0.826 mmol) and compound 7 (247 mg, 1.10 mmol). It was stirred at room temperature for 16h. It was diluted with water (20 ml) and extracted with EtOAc (50 ml), washed with satd NaHCO₃ (20 ml), brine (10 ml), dried over Na2SO4 and concentrated under reduced pressure. It was purified by column chromatography (3% MeOH-DCM) to afford compound **9** (180 mg, 74%) as gum.

Compound **9** (180 mg, 0.407 mmol) was taken in EtOH (20 ml) and was degassed with argon for 10 minutes. Pd/C (110 mg, 10% moist) was added and the mixture was subjected to hydrogenation in a Parr vessel at 50psi for 16h. It was filtered through celite, concentrated under reduced pressure and purified by preparative TLC (4% MeOH-DCM) to afford MMV689260 (120 mg, 83 %) as colourless gum. ¹H NMR (400 MHz, DMSO-*d₆*) δ 0.79 (d, 6 H), 0.95 (d, 3H), 2.31 (t, 2H), 2.79 (t, 2H), 2.93-2.96 (m, 1H), 3.14-3.18 (m, 2H), 3.27-3.32 (m, 1H), 3.72 (d, 1H), 3.81-3.86 (m, 1H), 4.46 (t, 1H, -OH), 5.38 (d, 1H, -OH), 7.10-7.14 (m, 2H), 7.21-7.28 (m, 2H), 7.68-7.70 (m, 2H).

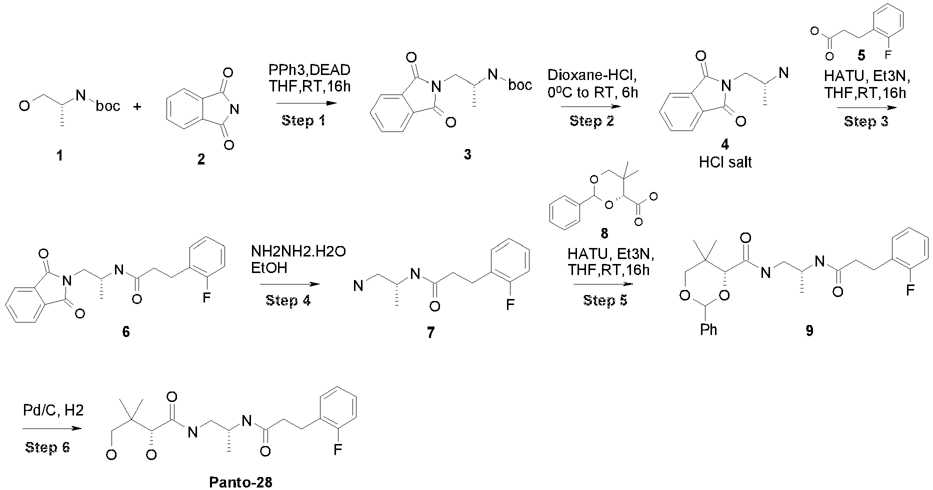

### Example 2: stability of pantothenamide analogues in vitro

Conventional pantothenamides are hydrolysed by pantetheinases in body fluids as shown before (Jansen et al 2013) thereby losing their antimicrobial activity (Jansen et al 2013, Spry et al 2013, PLoS One 8:e54974). Bioisosteric pantothenamides retained their in vitro antimicrobial activity in the presence of serum, as will be shown in the next examples. To examine the stability of analogues in vitro, CXP18.3-002 and its bioisostere CXP18.6-006 were incubated for 16 h at room temperature in PBS with 10% fetal bovine serum as a source of pantetheinase activity. Samples were taken and analyzed by LC-MS using a Shimadzu LC10ATvp HPLC coupled to a Shimadzu LCMS2010A mass spectrometer. No appreciable degradation of the bioisosteric compound was noted. When the parent pantothenamide CXP18.3-002 was incubated according to this protocol, the compound was completely hydrolysed. The hydrolysis of the pantothenamides by fetal bovine serum could be inhibited by the addition of RR6, a potent inhibitor of pantetheinase activity, as described before (Jansen en al 2013). Figure 1 shows the chromatograms of CXP18.6-002 and CXP18.6-006.

### Example 3: Stability in vivo

To address the question if increased stability would also lead to improved bioavailability in vivo, a pharmacokinetic study was performed. Briefly, the pantothenamides N5-Pan (an antibacterial compound) and CXP18.3-002 (an antimalarial compound), and their corresponding inverted amides CXP18.6-013 and CXP18.6-006 were administered to rats at 50 mg/kg dissolved in saline. Blood samples were collected from 3 rats before the experiment and at 0.25, 0.5, 1, 2, 4, 6, 8, 12 and 24 h. The concentrations of the compounds were analyzed by LC-MS/MS (AB Sciex API-4000 system). Data were analyzed by the Non-compartmental analysis tool of Phoenix WinNonlin (Version 6.3). Table 2 lists the pharmacokinetic parameters.

**Table 2 . Pharmacokinetic parameters of pantothenamides and cognate analogues.**

| Compound | Tₘₐₓ (h) | Cₘₐₓ (ng/mL) | AUC_{inf} (hr*ng/mL) | T_{1/2} (h) |
|---|---|---|---|---|
| N5-Pan | NC | NC | NC | NC |
| CXP18.3-002 | 0.5 | 5 | NC | NC |
| CXP18.6-013 | 0.33 | 12073 | 11551 | 5.9 |
| CXP18.6-006 | 0.33 | 3364 | 6622 | 3.6 |

| | | | | |
|---|---|---|---|---|
| NC: not calculated as levels were too low/below the detection limit | | | | |

As expected the conventional pantothenamides N5-Pan and CXP18.3-002 could not or hardly be detected following oral administration. For CXP18.3-002, reliable data for 3 rats were obtained only at 0.5 h. Pharmacokinetic parameters could not be determined as the compound was below the limit of detection at most timepoints measured. The corresponding inverted amides showed excellent bioavailability and stability as witnessed by half-lives of 5.9 and 3.6 h, calculated from the elimination phase.

### Example 4: Activity against the asexual blood stages of the human malaria parasite Plasmodium falciparum

Compounds were tested on their ability to inhibit replication of the human malaria parasite Plasmodium falciparum. To this end, Plasmodium falciparum NF54 parasites were cultured in RPMI1640 medium supplemented with 367 µM hypoxanthine, 25 mM HEPES, 25 mM sodiumbicarbonate and 10% human type A serum in an automated large-scale culture system as described before (Transactions of the Royal Society of Tropical Medicine and Hygiene, 1982. 76: p. 812-818). Compounds were diluted in DMSO and subsequently in culture medium to reach a DMSO concentration of 0.2%. 50 µl of diluted compound was transferred to a black 96 well plate. A culture of *P. falciparum* strain NF54 malaria parasites was diluted to a parasitaemia of 0.5% and a hematocrit of 3%. 50 µl of this diluted culture was added to each well in the 96 well plate. The outermost wells of the plate were filled with sterile water to prevent evaporation. The established antimalarial dihydroartemisinin (DHA) in DMSO acted as a positive control (MIN signal), and 0.1% DMSO acted as a negative control (MAX signal). The plates were incubated for 72 at 37°C, 3% O₂, 5% CO₂, 93% N₂. Changes in parasite replication relative to the controls were monitored using the DNA-marker SYBR Green as described previously (Antimicrob Agents Chemother 48:1803-6). Data were analyzed by logistic regression using the formula: Y=Bottom + (Top-Bottom)/(1+10^((LogIC50-X)*HillSlope)) and a least squares method to find the best fit.

Figure 2 shows the growth inhibition curves of CXP18.6-017 and CXP18.6-052. Using similar methods, IC50 values were determined for a variety of inverted pantothenamides, as listed in table 1 (see section: 'Detailed description of the invention')

### Example 5: Competition with pantothenate

To study their interaction with pantothenate-dependent pathways, asexual replication assays were performed with CXP18.6-017, CXP18.6-026 and CXP18.6-052 in the presence of increasing amounts pantothenate. When combining a pantothenamide analogue and pantothenate in the same well the conditions were otherwise similar to the single compound assays described above, however the final DMSO concentration was 0.2%, which was also adjusted for the positive and negative controls. The data shown in figure 3 indicate that the IC50 for all three compounds tested increased with increasing amounts of pantothenate in the culture medium. Schild regression analysis revealed a linear correlation between the log dose ratio -1 and the log of the pantothenate concentration for compound CXP18.6-052, indicating competitive antagonism on a presumed shared binding site (figure 3D). The Schild regression curves for CXP18.6-017 and CXP18.6-026 did not reveal a linear correlation (not shown), suggesting these compounds do not show competitive antagonism on a pantothenate dependent pathway, but may act in a non-competitive mode of action.

### Example 6: Activity against the sexual blood stages of the human malaria parasite Plasmodium falciparum

Compounds were tested for their ability to affect the viability of gametocytes, the sexually committed blood stages of *P. falciparum* that underlie transmission to the mosquito vector. To this end, two different assays were performed. The first assay monitored pLDH activity as a measure of viability of gametocytes (J Antimicrob Chemother. 2013 Sep;68(9):2048-58). The second assay monitored the ability of gametocytes to form a female gamete by detection of the gamete-specific marker Pfs25. Plasmodium falciparum NF54 parasites were cultured in RPMI1640 medium supplemented with 367 µM hypoxanthine, 25 mM HEPES, 25 mM sodiumbicarbonate and 10% human type A serum in an automated large-scale culture system as described before (Transactions of the Royal Society of Tropical Medicine and Hygiene, 1982. 76: p. 812-818). Cultures were treated with 50 mM N-acetyl-D-glucosamine from day 4 till day 9 to kill asexual parasites. On day 11 gametocytes were isolated by discontinuous 63% Percoll gradient centrifugation. Compounds were diluted in DMSO and subsequently in culture medium to reach a DMSO concentration of 0.2%. To measure the effect of compounds of pLDH activity, gametocytes were seeded at a density of 3.5x10⁴ gametocytes per well in a black 384 well plate in 30 µl of culture medium, and 30 µl of diluted compound was added. Following a 72 hour incubation at 37°C, 98% humidity, 93% N₂, 4% CO₂ and 3% O₂, gametocyte viability was measured by a modified plasmodium lactate dehydrogenase assay by using resazurin as a substrate as previously described (Nature, 2013. 504(7479): p. 248-53). Assays were performed in quadruplicate (two different plates with two different gametocyte batches) and all plates included controls MIN (1 µM DHA), MAX (0.1% DMSO) and a serial dilution of reference compound dihydroartemisinin (DHA). The data presented in Figure 4A indicate that CXP17.6-017, CXP18.6-26 and CXP18.6-052 dose dependently reduced gametocyte viability, as evidenced by a reduction in pLDH activity. Whereas CXP17.6-017, CXP18.6-26 inhibited gametocyte viability to the same extent as the reference compound DHA, CXP18.6-052 showed a partial effect with a maximum inhibition of ∼50%. Additional compounds were tested in a similar manner, and IC50 values are presented in table 3.

To monitor compound effects on the ability of a gametocyte to form a female gamete, day 11 (stage IV) gametocytes were seeded at a density of 4x10⁵ gametocytes per well in a V-bottom plate in 50 µl culture medium. 50 µl of diluted compound was added to the gametocytes (0.1% DMSO final concentration) and plates were incubated for 72 hours at 37°C, 98% humidity, 93% N₂, 4% CO₂ and 3% O₂. Subsequently, gametogenesis was induced by adding xanthurenic acid to a final concentration of 40 µM and plates were incubated for 16 hours at 20°C. Gametes were detected in a homogeneous proximity immunoassay on basis of Pfs25 expression by use of AlphaLISA technology (Perkin Elmer). All steps were performed at 20°C. Gametes were washed twice with PBS, lysed for 15 min in 10 µl 10 mM Tris pH 8.0 / 1% laurylsarcosyl / ImM phenylmethanesulfonyl fluoride and combined with 10 µl 5 nM biotinylated mouse anti-Pfs25 monoclonal antibody 32F6244 in a white 384 well optiplate (Perkin Elmer). After 1 hour incubation 15µl of 33 µg/ml acceptorbeads coated with mouse anti-Pfs25 monoclonal antibody 32F81 were added. After 1 hour incubation, 15 µl 33 µg/ml Streptavidin-conjugated donorbeads were added. Finally, after 1 hour incubation luminescence signals were monitored at the 615 nm emission wavelength following excitation at 680 nm using a fluorometer (Biotek Synergy 2). All compounds were tested in duplicate (24hr) or triplicate (1 and 72hr). Controls included MIN (0.1 µM epoxomicin) and MAX (0.1% DMSO). Data were analyzed by logistic regression using the formula: Y=Bottom + (Top-Bottom)/(1+10^((LogIC50-X)*HillSlope)) and a least squares method to find the best fit. CXP18.6-017 and CXP18.6-026 potently inhibited the female gametogenesis capacity of gametocytes, with IC50 values of 37 and 13 nM, respectively (Figure 4B). In contrast, CXP18.6-052 was less potent and inhibited gametogenesis with an IC50 value of 1974 nM. Additional compounds were tested in a similar manner, and IC50 values are presented in table 3.

**Table 3. Compound activities against P. falciparum gametocytes**

| Compound | Gametocyte viability (pLDH) IC50 (µM) | Gametocyte gametogenesis capacity (Pfs25) IC50 (µM) |
|---|---|---|
| CXP18.6-006 | >10 | 0.478 |
| CXP18.6-017 | 0.1 | 0.036 |
| CXP18.6-026 | 0.17 | 0.013 |
| CXP18.6-046 | >10 | 0.401 |
| CXP18.6-047 | >10 | 0.168 |
| CXP18.6-064 | 0.042 | 0.017 |

### Example 7: Inhibition of transmission of the human malaria parasite Plasmodium falciparum

To assess the effect of a reduction in gametocyte viability and or capacity to form a gamete on actual transmission of the parasites, Standard Membrane Feeding Assays (SMFA) were performed using a transgenic P. falciparum NF54 strain that expresses a luciferase reporter gene from a hsp70 promoter. Parasite cultures showing ∼3% mature gametocytes were checked for the ability to show exflagellation after addition of xanthurenic acid to a final concentration of 40 µM. Infectivity of cultures showing positive exflagellation was tested in a 'prefeed' experiment as described previously (Antimicrob Agents Chemother, 2012. 56(7): p. 3544-8). Cultures showing development of retorts and ookinetes in the mosquito midgut were selected for testing of compounds. In the indirect mode of the assay, gametocytes were pre-incubated with compound prior to mosquito feeding. To this end, 40 µl compound diluted in parasite culture medium was added to 360 µl of parasite culture and incubated for 24 hrs at 37°C in Eppendorf tubes (0.1% DMSO final concentration). Hereafter, 300 µl was added to 180 µl of packed red blood cells and centrifuged for 20 seconds at 10,000 g. After carefully aspirating the supernatant, 200 µl of human serum type A was added to the pellet. Finally, 300 µl was immediately injected into an individual membrane-covered minifeeder, where 50 Anopheles stephensi mosquitoes of 1-to-5-days old were allowed to feed for 10 minutes and non-fed mosquitoes were removed from the cage. Eight days after feeding, 20 mosquitoes were sacrificed and parasite luciferase activity was measured as described previously (Stone, J Infect Dis. 2014 in press). For the direct SMFA, 300 µl parasite culture was added to 180 µl red blood cells, centrifuged for 20 seconds at 10,000 g and the supernatant was carefully aspirated. Meanwhile, 10 µl of compound diluted in incomplete medium was added to 490 µl human serum type A (0.1% DMSO final). 200 µl of this compound containing human serum was added to the pellet, which was subsequently used for the SMFA as described above. For dose response analyses, compounds were diluted in DMSO with subsequent dilution in culture medium to a final DMSO concentration of 0.1%. Controls included vehicle (0.1% DMSO) and DHA at its approximate IC90 (10 µM). Data were analyzed by logistic regression using the formula: Y=Bottom + (Top-Bottom)/(1+10^((LogIC50-X)*HillSlope)) and a least squares method to find the best fit.

In comparison with the DMSO control, CXP18.6-017 reduced luciferase intensity in the direct mode of the assay, indicating it inhibits gametogenesis and/or early sporogonic development in the mosquito midgut (Figure 5A). The inhibitory effect was stronger in the indirect mode of the assay, where gametocytes were pre-exposed to compound prior to mosquito feeding. These data are in line with the gametocyte viability and gametogenesis assays described above that indicated a gametocytocidal action of CXP18.6-017. The compound was tested in full dose response in the indirect mode of the SMFA, revealing an IC50 of 20 nM on the average luciferase intensity (Figure 5B). In terms of parasite prevalence, or the percentage of mosquitoes showing luciferase signals above background levels, the compound inhibited mosquito infection with an IC50 of 178 nM (Figure 5C). Likewise, CXP18.6-026 inhibited luciferase intensity and the number of infected mosquitoes with IC50 values of 107 and 370 nM, respectively (Figures 5D&E).

### Example 8: In vivo antimalarial activity of pantothenamide analogues

Female mice (C57 Black 6, 9-10 weeks of age at the start of the experiment) and female rats (Wistar, 150-200 grams at the beginning of the experiment) were housed under standard conditions and experiments were approved by the institutional ethical committee on animal care and experimentation of Radboud University Medical Centre (Nijmegen).

To test our compounds in an *in vivo* malaria model we used a well described rodent malaria model (Zuckerman and Yoeli, J Infect Dis. 94, 225-36). First, donor mice were infected by 0.2 ml cryopreserved infected blood stocks containing the transgenic *Plasmodium berghei* strain 676cl1 (PbGFP-Luc). After four days the mice were sacrificed and their blood was collected. The parasitemia in the blood from the mice was calculated as percentage of infected red blood cells (RBC) by Giemsa-stained blood smears. Infected blood was diluted to 5x10⁷ parasites per ml and 0.2 ml was i.p. injected in rats (inoculation of 10⁷ parasites per rat). Treatment with chloroquine (6 mg/kg), CXP18.6-017 (100 mg/kg) and CXP18.6-052 (100 mg/kg) started 5 days post inoculation and where given orally three times a day until day eight. From day 5 blood samples were collected and parasitemia was determined by analysis of Giemsa-stained blood smears. The infection and treatment regimes were well tolerated by the animals.

Figure 6 shows the parasitemia from day 5 to day 8. As expected, the positive control drug, chloroquine, reduced parasitemia to nearly undetectable levels within 3 days. CXP18.6-017 reduced parasitemia with similar kinetics as found for chloroquine. CXP18.6-052 showed a significant, albeit partial, reduction of parasitemia. In addition to manual counting of parasitemia, the parasite numbers were also quantitated by luciferase activity in whole blood samples, which gave essentially similar results (not shown).

### Example 9: Bacterial growth assays and MIC determination

Strains of Escherichia coli, Staphylococcus aureus and Staphylococcus epidermidis, were grown overnight in 1% Bacto tryptone medium (BD, Sparks, MD). Streptococcus pyogenes was grown overnight in Bacto Todd Hewitt Broth medium (BD, Sparks, MD). Cultures were then diluted 1:1,000 in fresh assay medium supplemented with or without 20% fetal bovine serum (FBS; HyClone, Celbio, Logan, UT) and added to 96-well sterile enzyme-linked immunosorbent assay (ELISA) plates (100 µl per well). The compounds to be tested were diluted in the medium without serum and added to the bacteria (100 µl per well). Plates were incubated at 37°C, and bacterial growth was monitored over time by reading the optical density at 490 nm using a microplate reader (model 450; Bio-Rad Laboratories Inc., Hercules, CA).

Streptococcus pneumoniae strains were grown to mid-log phase (OD620 of 0.3) and stored in 15% glycerol at 80°C till further use. Minimal inhibitory concentration (MIC) of antibiotics was determined according to standard procedures. Bacteria were diluted 10-fold in Mueller-Hinton medium (BD, Sparks, MD) and added to 96-well sterile ELISA plates (150 µl per well). The compounds to be tested were diluted in the medium without serum and added to the bacteria (150 µl per well). Plates were incubated overnight at 37°C in 5% CO2. The MIC was defined as the lowest concentration of compound at which no growth was visible with the naked eye.

As shown before (Jansen et al 2013) pantothenamides were found to have poor antibiotic activity when bacteria were cultured in the presence of serum. Table 4 shows that the MIC values of the pantothenamides N7-Pan and N9-Pan towards S.aureus in the presence of 10% serum were > 256 µg/ml, whereas in the absence of serum these compounds are potent antibiotics with MIC values of 0.5 µg/ml. The corresponding inverted amides, CXP18.6-012 and CXP18.6-014 retained activity when tested in the presence of serum. In general, the MIC values of the inverted amides were higher than those of the parent compounds, indicating a loss of potency, although this varied depending on the species tested. In some cases, a significant loss or gain of activity was noted for the inverted pantothenamide compared to the parent compound. CXP18.6-012 combined with *S.pneumoniae* is an example of loss of activity. CXP18.6-013 provides an example of gain of antimicrobial activity when used for *S.epidermidis.*

**Table 4. Antibacterial activities of pantothenamides and corresponding inverted amides.**

| Organism | serum | pantothenamide | MIC | inverted pantothenamide | MIC |
|---|---|---|---|---|---|
| S.aureus | - | N5-Pan | 8 | CXP18.6-013 | 32 |
| S.epidermidis | - | N5-Pan | 32 | CXP18.6-013 | 1 |
| S.pneumoniae | - | N5-Pan | 0.25 | CXP18.6-013 | 4 |
| S.pyogenes | - | N5-Pan | nd | CXP18.6-013 | 2 |
| E.coli | - | N5-Pan | 64 | CXP18.6-013 | >256 |
| S.aureus | - | N7-Pan | 0.5 | CXP18.6-012 | 1 |
| S.aureus | + | N7-Pan | >256 | CXP18.6-012 | 4 |
| S.epidermidis | - | N7-Pan | 0.5 | CXP18.6-012 | 16 |
| S.pneumoniae | - | N7-Pan | 2 | CXP18.6-012 | >32 |
| S.pyogenes | - | N7-Pan | 2 | CXP18.6-012 | 32 |
| E.coli | - | N7-Pan | 128 | CXP18.6-012 | >256 |
| S.aureus | - | N9-Pan | 0.5 | CXP18.6-014 | 1 |
| S.aureus | + | N9-Pan | >256 | CXP18.6-014 | 1 |
| S.epidermidis | - | N9-Pan | 0.25 | CXP18.6-014 | 1 |
| S.pneumoniae | - | N9-Pan | nd | CXP18.6-014 | >32 |
| S.pyogenes | - | N9-Pan | 16 | CXP18.6-014 | 32 |
| E.coli | - | N9-Pan | >256 | CXP18.6-014 | >256 |
| S.aureus | - | CXP14.19-014 | 1 | CXP18.6-017 | 2 |
| S.epidermidis | - | CXP14.19-014 | 2 | CXP18.6-017 | 32 |
| S.pneumoniae | - | CXP14.19-014 | nd | CXP18.6-017 | >32 |
| S.pyogenes | - | CXP14.19-014 | 8 | CXP18.6-017 | 8 |
| E.coli | - | CXP14.19-014 | >256 | CXP18.6-017 | >256 |

## Claims

1. Compound selected from the group consisting of pantothenamide analogues represented by formula (I) and pharmaceutically acceptable salts and esters thereof: 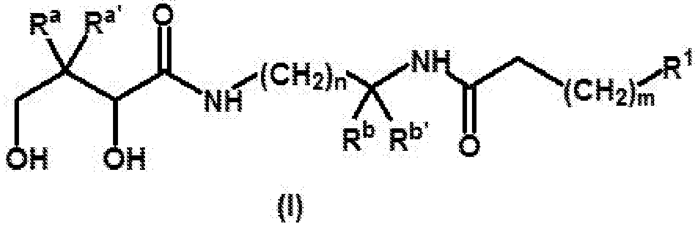 wherein
n = 1, 2, 3 or 4
m = 0, 1, 2, 3 or 4
moiety
R^{a} and R^{a'} are independently selected from the group consisting of (C₁-C₆)alkyl, (C₁-C₆)alkenyl, (C₁-C₆)alkynyl, branched (C₃-C₆)alkyl, O(C₁-C₅)alkyl and CH₂-O-C₁-C₄ alkyl; or
R^{a} and R^{a'} are joined together to form a single moiety selected from the group consisting of-(C₃-C₆)alkyl-, -(C₁-C₂)alkyl-O-(C₁-C₂)alkyl- and -(C₁-C₂)alkyl-NR-(C₁-C₂)alkyl-, wherein R represents hydrogen or C₁-C₆ alkyl;
R^{b} and R^{b'} are independently selected from the group consisting of hydrogen, (C₁-C₆)alkyl, (C₁-C₆)alkenyl, (C₁-C₆)alkynyl, branched (C₃-C₆)alkyl, (C₃-C₆)cycloalkyl and aryl; and
R¹ represents:
- an aliphatic or heteroaliphatic moiety selected from the group consisting of C₁-C₁₀ alkyl, C₂-C₁₀-alkenyl, C₂-C₁₀-alkynyl, C₁-C₅-alkyl-C₃-C₆ cycloalkyl, (C₁-C_{y})-O-(C₁-Cₓ) and (C₁-C_{y})-S-(C₁-Cₓ), wherein y and x each represent an integer in the range of 0-7 and wherein y+x≤7, each moiety optionally being substituted with 1-3 substituents independently selected from halo, C₁-C₆ alkyl, C₃-C₆ cylcoalkyl, phenyl, C₁-C₆ haloalkyl, C₁-C₆ alkoxy and CH₂-O-C₁-C₆alkyl
- an aromatic moiety selected from the group consisting of phenyl and naphtyl, each optionally substituted with 1-3 substituents independently selected from halo, C₁-C₆ alkyl, C₃-C₆ cylcoalkyl, phenyl, C₁-C₆ haloalkyl, C₁-C₆ alkoxy and CH₂-O-C₁-C₆alkyl; or
- a heteroaromatic moiety selected from the group consisting of thienyl, benzothienyl, furyl, benzopyranyl, pyrol, indolyl, pyran, pyrimidine, triazine, pyridyl, quinolyl, isoquinolyl, isoxazole, oxazole, pyrazole, thiazole, imidazole, triazole, oxadiazole, thiadiazole and tetrazole, each optionally substituted with 1-3 substituents independently selected from halo, C₁-C₆ alkyl, C₃-C₆ cylcoalkyl, phenyl, C₁-C₆ haloalkyl, C₁-C₆ alkoxy and CH₂-O-C₁-C₆alkyl.

2. Compound according to claim 1, wherein R^{a} and R^{a'} both represent methyl.

3. Compound according to claim 1, wherein one of R^{b} and R^{b'} represents methyl or ethyl, preferably methyl, and the other one of R^{b} and R^{b'} represents hydrogen.

4. Compound according to claim 1, wherein R^{b} and R^{b'} both represent hydrogen.

5. Compound according to claim 1, wherein n = 1 or 2, preferably n = 1.

6. Compound according to claim 1 or 2, wherein m = 1 or 2, preferably m = 1.

7. Compound according to any one of the preceding claims, wherein m = 1 and R¹ represents C₁-C₁₀ alkyl.

8. Compound according to any one of the preceding claims, wherein m=1 or 2 and R¹ represents phenyl, optionally substituted with one or two substituents selected from the group consisting of methyl, ethyl, propyl, t-butyl, methoxy, ethoxy, chloro, fluoro and trifluoromethyl.

9. Compound according to any one of the preceding claims, wherein m=1 or 2 and R¹ represents a group as defined in claim 1, which is also a C₅ or C₆ heteroaryl comprising one heteroatom selected from oxygen, nitrogen and sulphur, optionally substituted with a substituent selected from the group consisting of methyl, ethyl, propyl, t-butyl, methoxy, ethoxy, chloro, fluoro and trifluoromethyl.

10. Compound according to claim 1, selected from the group consisting of CXP18.6-052; CXP18.6-017; CXP18.6-064; CXP18.6-046; CXP18.6-047; CXP18.6-018; CXP18.6-069; CXP18.6-006; CXP18.6-043; CXP18.6-026; CXP14.18-019; CXP18.6-028; CXP18.6-042; CXP18.6-012; CXP14.18-038; CXP18.6-057; CXP18.6-019; CXP18.6-056; CXP18.6-008; CXP18.6-027; CXP14.18-034; CXP18.6-013; CXP14.18-010; CXP14.18-008; CXP14.18-016; CXP14.18-028; CXP18.6-022; CXP14.18-037; CXP18.6-014; CXP14.18-035; CXP14.18-005; CXP14.18-014; CXP14.18-007; CXP14.18-012; CXP18.6-055; CXP14.18-020; CXP18.6-048; CXP18.6-007; CXP18.6-038; CXP18.6-009; CXP18.6-010, MMV689258, MMV689260 and pharmaceutically acceptable salts and esters thereof, wherein said compounds are defined as follows:
| Compound | Structure |
|---|---|
| CXP18.6-052 | 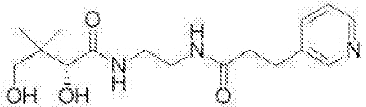 |
| CXP18.6-017 | 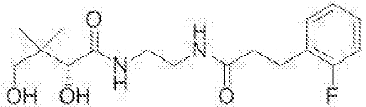 |
| CXP 18.6-064 | 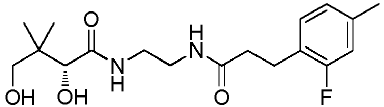 |
| CXP 18.6-046 | 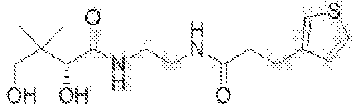 |
| CXP18.6-047 | 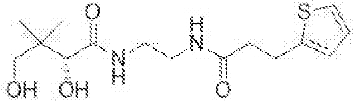 |
| CXP18.6-018 | 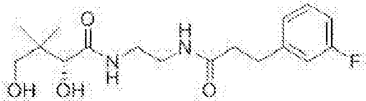 |
| CXP 18.6-069 | 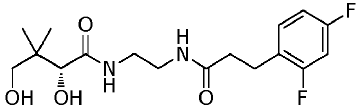 |
| CXP 18.6-006 | 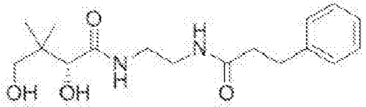 |
| CXP18.6-043 | 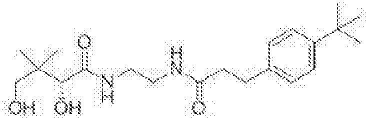 |
| CXP 18.6-026 | 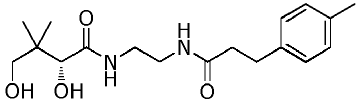 |
| CXP14.18-019 | 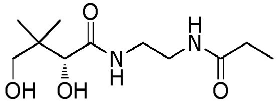 |
| CXP18.6-028 | 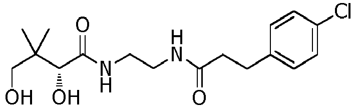 |
| CXP18.6-042 | 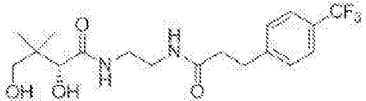 |
| CXP18.6-012 | 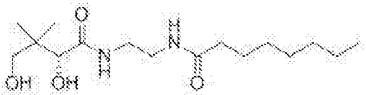 |
| CXP14.18-038 | 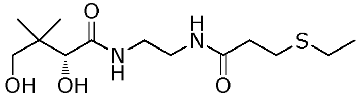 |
| CXP18.6-057 | 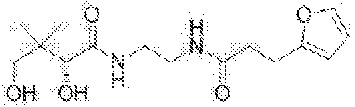 |
| CXP18.6-019 | 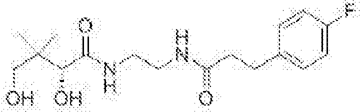 |
| CXP18.6-056 | 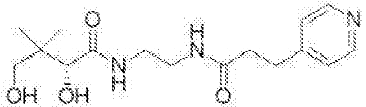 |
| CXP18.6-008 | 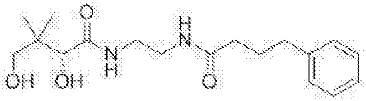 |
| CXP18.6-027 | 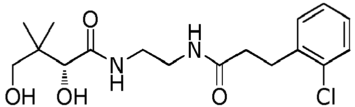 |
| CXP14.18-034 | 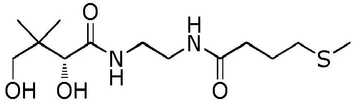 |
| CXP18.6-013 | 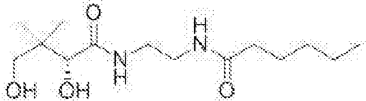 |
| CXP14.18-010 | 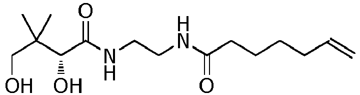 |
| CXP14.18-008 | 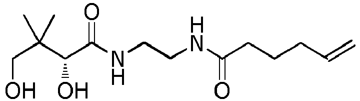 |
| CXP14.18-016 | 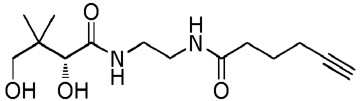 |
| CXP14.18-028 | 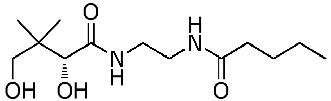 |
| CXP 18.6-022 | 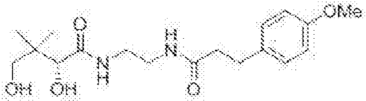 |
| CXP14.18-037 | 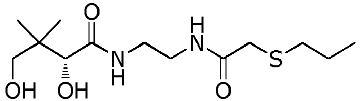 |
| CXP18.6-014 | 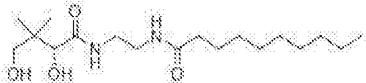 |
| CXP14.18-035 | 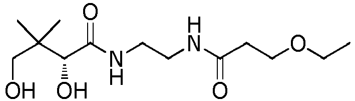 |
| CXP14.18-005 | 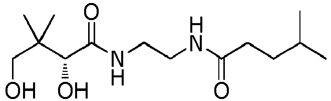 |
| CXP14.18-014 | 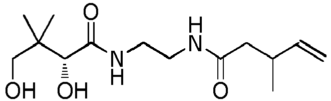 |
| CXP14.18-007 | 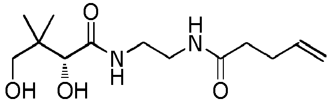 |
| CXP14.18-012 | 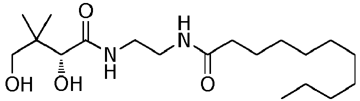 |
| CXP18.6-055 | 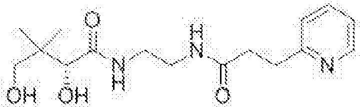 |
| CXP14.18-020 | 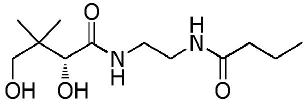 |
| CXP18.6-048 | 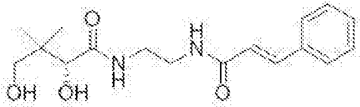 |
| CXP 18.6-007 | 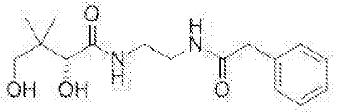 |
| CXP18.6-038 | 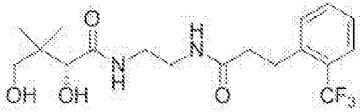 |
| CXP 18.6-009 | 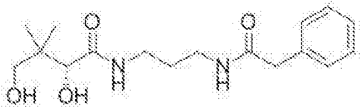 |
| CXP18.6-010 | 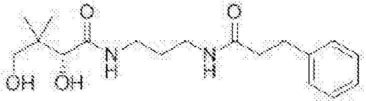 |
| MMV689258 | 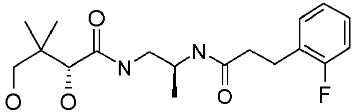 |
| MMV689260 | 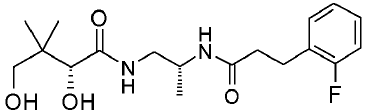 |

11. Pharmaceutical composition comprising a compound as defined in any one of claims 1-10 and a pharmaceutically acceptable excipient.

12. A compound as defined in any one of claims 1-10 for use in a method of treating or preventing a microbial infection in a human or animal subject in need thereof, said method comprising administering to said subject a compound as defined in any one of claims 1-10.

13. A compound for use according to claim 12, wherein said microbial infection is selected from a bacterial infection, a fungal infection and a protozoan infection.

14. A compound as defined in any one of claims 1-10 for use in a method of treating or preventing a disease in a human or animal subject, said method comprising administering to said subject a compound as defined in any one of claims 1-10, wherein said disease is a disease caused by protozoa, preferably by protozoa species selected from the genus of Plasmodium, Trypanosoma, Giardia, Cryptosporidium, Amoeba, Toxoplasma, Trichomonas or Leishmania

15. A compound as defined in any one of claims 1-10 for use in a method of treating or preventing a disease in a human or animal subject, said method comprising administering to said subject a compound as defined in any one of claims 1-10, wherein said disease is selected from the group consisting of Malaria, Amoebiasis, Giardiasis, Toxoplasmosis, Cryptosporidiosis, Trichomoniasis, Chagas disease, Leishmaniasis, Sleeping Sickness, Dysentery, Acanthamoeba Keratitis, Primary Amoebic Meningoencephalitis.

## Patentansprüche

1. Verbindung, ausgewählt aus der Gruppe bestehend aus Pantothenamidanaloga, die dargestellt sind durch Formel (I), und pharmazeutisch annehmbaren Salzen und Estern derselben: 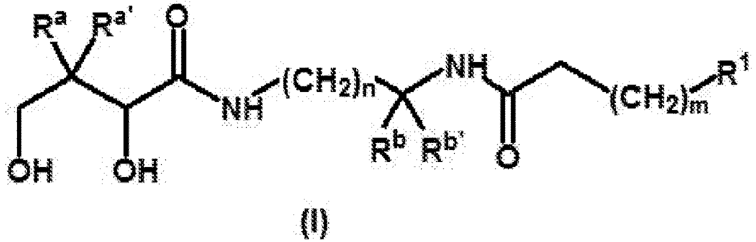 wobei
n = 1, 2, 3, oder 4 ist,
m = 0, 1, 2, 3 oder 4 ist,
Reste R^{a} und R^{a'} unabhängig ausgewählt sind aus der Gruppe bestehend aus (C₁-C₆)-Alkyl, (C₁-C₆)-Alkenyl, (C₁-C₆)-Alkinyl, verzweigtem (C₃-C₆)-Alkyl, O(C₁-C₅)-Alkyl und CH₂-O-C₁-C₄-Alkyl; oder R^{a} und R^{a'} miteinander verknüpft sind, um einen einzelnen Rest zu bilden, ausgewählt aus der Gruppe bestehend aus-(C₃-C₆)-Alkyl-, -(C₁-C₂)-Alkyl-O-(C₁-C₂)-Alkyl- und -(C₁-C₂)-Alkyl-NR-(C₁-C₂)-Alkyl-, wobei R Wasserstoff oder C₁-C₆-Alkyl darstellt;
R^{b} und R^{b'} unabhängig ausgewählt sind aus der Gruppe bestehend aus Wasserstoff, (C₁-C₆)-Alkyl, (C₁-C₆)-Alkenyl, (C₁-C₆)-Alkinyl, verzweigtem (C₁-C₆)-Alkyl, (C₃-C₆)-Cycloalkyl und Aryl; und R¹ darstellt:
- einen aliphatischen oder heteroaliphatischen Rest ausgewählt aus der Gruppe bestehend aus C₁-C₁₀-Alkyl, C₂-C₁₀-Alkenyl, C₂-C₁₀-Alkinyl, C₁-C₅-Alkyl-C₃-C₆-Cycloalkyl, (C₁-C_{y})-0-(C₁-Cₓ) und (C₁-C_{y})-S-(C₁-Cₓ), wobei y und x jeweils eine ganze Zahl im Bereich von 0 bis 7 aufweisen und y+x≤7 ist, wobei jeder Rest optional substituiert ist mit 1-3 Substituenten, die unabhängig ausgewählt sind aus Halogen, C₁-C₆-Alkyl, C₃-C₆-Cycloalkyl, Phenyl, C₁-C₆-Halogenalkyl, C₁-C₆-Alkoxy und CH₂-O-C₁-C₆-Alkyl,
- einen aromatischen Rest ausgewählt aus der Gruppe bestehend aus Phenyl und Naphtyl, jeweils optional substituiert mit 1-3 Substituenten, die unabhängig ausgewählt sind aus Halogen, C₁-C₆-Alkyl, C₃-C₆-Cycloalkyl, Phenyl, C₁-C₆-Halogenalkyl, C₁-C₆-Alkoxy und CH₂-O-C₁-C₆-Alkyl; oder
- einen heteroaromatischen Rest, ausgewählt aus der Gruppe bestehend aus Thienyl, Benzothienyl, Furyl, Benzopyranyl, Pyrol, Indolyl, Pyran, Pyrimidin, Triazin, Pyridyl, Chinolyl, Isochinolyl, Isoxazol, Oxazol, Pyrazol, Thiazol, Imidazol, Triazol, Oxadiazol, Thiadiazol und Tetrazol, jeweils optional substituiert mit 1-3 Substituenten, die unabhängig ausgewählt sind aus Halogen, C₁-C₆-Alkyl, C₃-C₆-Cycloalkyl, Phenyl, C₁-C₆-Halogenalkyl, C₁-C₆-Alkoxy und CH₂-O-C₁-C₆-Alkyl.

2. Verbindung nach Anspruch 1, wobei R^{a} und R^{a'} beide Methyl darstellen.

3. Verbindung nach Anspruch 1, wobei eines von R^{b} und R^{b'} Methyl oder Ethyl, bevorzugt Methyl, darstellt und der andere von R^{b} und R^{b'} Wasserstoff darstellt.

4. Verbindung nach Anspruch 1, wobei R^{b} und R^{b'} beide Wasserstoff darstellen.

5. Verbindung nach Anspruch 1, wobei n = 1 oder 2 ist, bevorzugt n = 1 ist.

6. Verbindung nach Anspruch 1 oder 2, wobei m = 1 oder 2, bevorzugt m = 1 ist.

7. Verbindung nach einem der vorangehenden Ansprüche, wobei m = 1 ist und R¹ C₁-C₁₀-Alkyl darstellt.

8. Verbindung nach einem der vorangehenden Ansprüche, wobei m = 1 oder 2 ist und R¹ Phenyl darstellt, optional substituiert mit einem oder zwei Substituenten ausgewählt aus der Gruppe bestehend aus Methyl, Ethyl, Propyl, t-Butyl, Methoxy, Ethoxy, Chlor, Fluor und Trifluormethyl.

9. Verbindung nach einem der vorangehenden Ansprüche, wobei m = 1 oder 2 ist und R¹ eine Gruppe wie definiert in Anspruch 1 darstellt, die ebenfalls ein C₅- oder C₆-Heteroaryl umfassend ein Heteroatom ausgewählt aus Sauerstoff, Stickstoff und Schwefel, ist, optional substituiert mit einem Substituenten ausgewählt aus der Gruppe bestehend aus Methyl, Ethyl, Propyl, t-Butyl, Methoxy, Ethoxy, Chlor, Fluor und Trifluormethyl.

10. Verbindung nach Anspruch 1, ausgewählt aus der Gruppe bestehend aus CXP18.6-052; CXP18.6-017; CXP18.6-064; CXP18.6-046; CXP18.6-047; CXP18.6-018; CXP18.6-069; CXP18.6-006; CXP18.6-043; CXP18.6-026; CXP14.18-019; CXP18.6-028; CXP18.6-042; CXP18.6-012; CXP14.18-038; CXP18.6-057; CXP18.6-019; CXP18.6-056; CXP18.6-008; CXP18.6-027; CXP14.18-034; CXP18.6-013; CXP14.18-010; CXP14.18-008; CXP14.18-016; CXP14.18-028; CXP18.6-022; CXP14.18-037; CXP18.6-014; CXP14.18-035; CXP14.18-005; CXP14.18-014; CXP14.18-007; CXP14.18-012; CXP18.6-055; CXP14.18-020; CXP18.6-048; CXP18.6-007; CXP18.6-038; CXP18.6-009; CXP18.6-010, MMV689258, MV689260 und pharmazeutisch annehmbaren Salzen und Estern derselben, wobei die Verbindungen wie folgt definiert sind:
| Verbindung | Struktur |
|---|---|
| CXP18.6-052 | 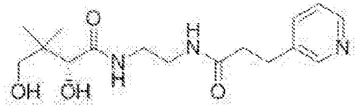 |
| CXP18.6-017 | 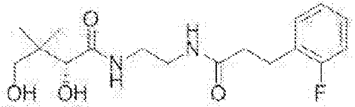 |
| CXP18.6-064 | 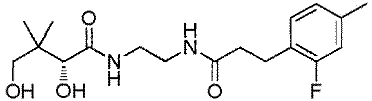 |
| CXP18.6-046 | 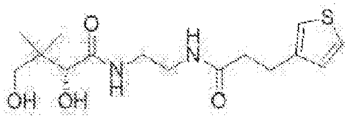 |
| CXP18.6-047 | 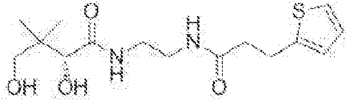 |
| CXP18.6-018 | 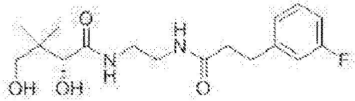 |
| CXP18.6-069 | 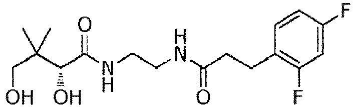 |
| CXP18.6-006 | 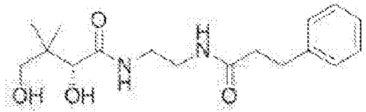 |
| CXP18.6-043 | 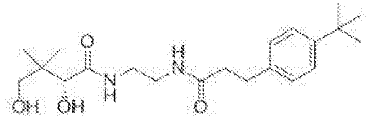 |
| CXP18.6-026 | 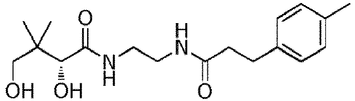 |
| CXP14.18-019 | 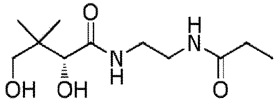 |
| CXP18.6-028 | 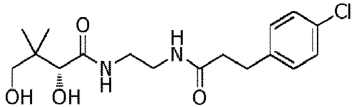 |
| CXP18.6-042 | 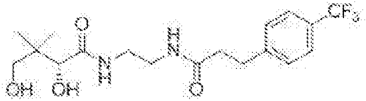 |
| CXP18.6-012 | 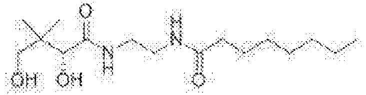 |
| CXP14.18-038 | 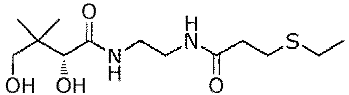 |
| CXP18.6-057 | 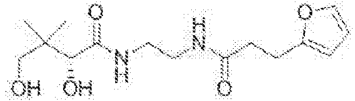 |
| CXP18.6-019 | 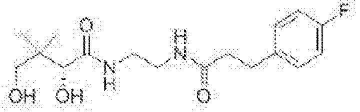 |
| CXP18.6-056 | 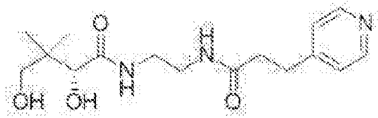 |
| CXP18.6-008 | 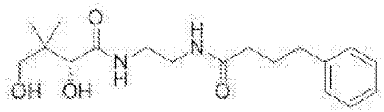 |
| CXP18.6-027 | 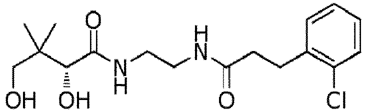 |
| CXP14.18-034 | 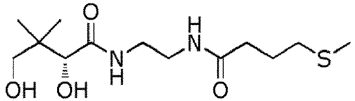 |
| CXP18.6-013 | 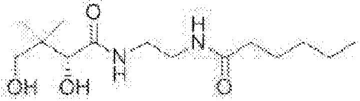 |
| CXP14.18-010 | 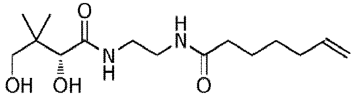 |
| CXP14.18-008 | 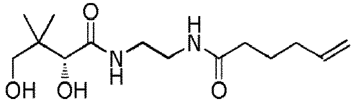 |
| CXP14.18-016 | 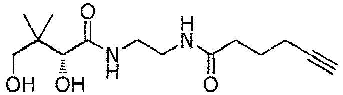 |
| CXP14.18-028 | 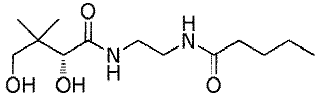 |
| CXP18.6-022 | 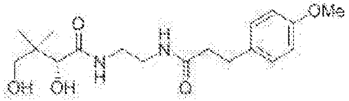 |
| CXP14.18-037 | 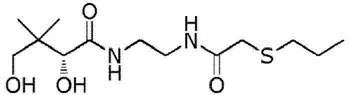 |
| CXP18.6-014 | 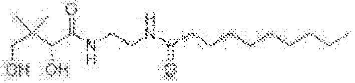 |
| CXP14.18-035 | 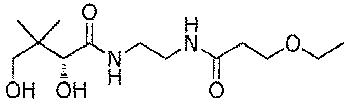 |
| CXP14.18-005 | 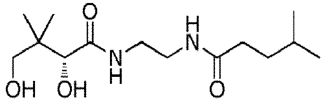 |
| CXP14.18-014 | 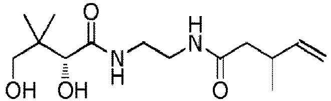 |
| CXP14.18-007 | 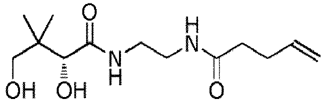 |
| CXP14.18-012 | 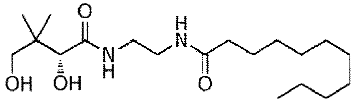 |
| CXP18.6-055 | 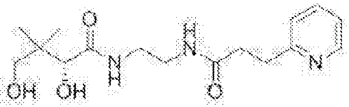 |
| CXP14.18-020 | 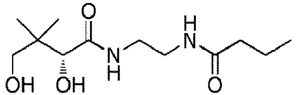 |
| CXP18.6-048 | 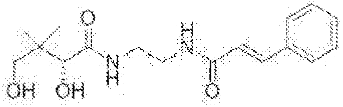 |
| CXP18.6-007 | 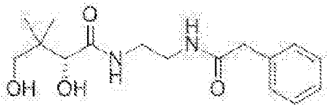 |
| CXP18.6-038 | 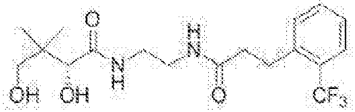 |
| CXP18.6-009 | 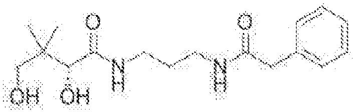 |
| CXP18.6-010 | 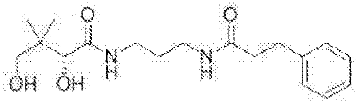 |
| MMV689258 | 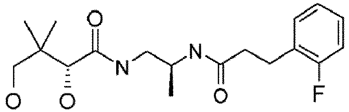 |
| MMV689260 | 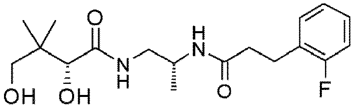 |

11. Pharmazeutische Zusammensetzung umfassend eine Verbindung, wie sie in einem der Ansprüche 1 bis 10 definiert, und einen pharmazeutisch annehmbaren Arzneistoffträger.

12. Verbindung, wie sie in einem der Ansprüche 1 bis 10 definiert ist, zur Verwendung in einem Verfahren zum Behandeln oder Verhindern einer mikrobiellen Infektion bei einem Menschen oder einem Tier, der bzw. das diese benötigt, wobei das Verfahren ein Verabreichen an den Menschen oder das Tier einer Verbindung, wie sie in einem Ansprüche 1 bis 10 definiert ist, umfasst.

13. Verbindung zur Verwendung nach Anspruch 12, wobei die mikrobielle Infektion ausgewählt ist aus einer bakteriellen Infektion, einer Pilzinfektion und einer Protozoaninfektion.

14. Verbindung, wie sie in einem der Ansprüche 1 bis 10 definiert ist, zur Verwendung in einem Verfahren zum Behandeln oder Verhindern einer Erkrankung bei einem Menschen oder einem Tier, wobei das Verfahren umfasst ein Verabreichen an den Menschen oder das Tier einer Verbindung, wie sie in einem der Ansprüche 1 bis 10 definiert ist, wobei die Erkrankung eine Erkrankung ist, die durch Protozoa verursacht wird, bevorzugt durch Protozoa-Spezies ausgewählt aus der Gattung Plasmodium, Trypanosoma, Giardia, Cryptosporidium, Amoeba, Toxoplasma, Trichomonas oder Leishmania.

15. Verbindung, wie sie in einem der Ansprüche 1 bis 10 definiert ist, zur Verwendung in einem Verfahren zum Behandeln oder Vermeiden einer Erkrankung bei einem Menschen oder einem Tier, wobei das Verfahren umfasst ein Verabreichen an den Menschen oder das Tier einer Verbindung, wie sie in einem der Ansprüche 1 bis 10 definiert ist, wobei die Erkrankung ausgewählt ist aus der Gruppe bestehend aus Malaria, Amöbenkrankheit, Giardiasis, Toxoplasmose, Cryptosporidiosis, Trichmoniasis, Chagas-Krankheit, Leishmaniase, Schlafkrankheit, Diarrhoe, Acanthamoebakeratitis, primäre amöbische Meningoenzephalitis.

## Revendications

1. Composé choisi dans le groupe constitué par des analogues de pantothénamide représentés par la formule (I) et des sels et esters pharmaceutiquement acceptables de ceux-ci : 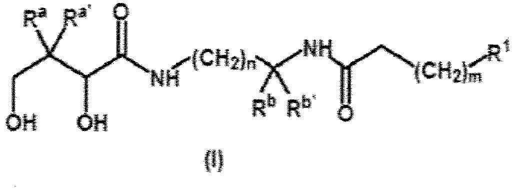 dans lequel
n = 1, 2, 3 ou 4
m = 0, 1, 2, 3 ou, 4
fragment
R^{a} et R^{a'} sont choisis indépendamment dans le groupe constitué par un alkyle en C₁₋₆, un alcényle en C₁₋C₆, un alcynyle en C₁₋C₆, un alkyle en C₃₋C₆ ramifié, un O-alkyle en C₁-C₅ et CH₂ et un CH₂-O-alkyle en C₁-C₄ ; ou R^{a} et R^{a'} sont reliés ensemble pour former un fragment unique choisi dans le groupe consistant en un -alkyle en C₃-C₆-, un -alkyle en C₁-C₂-O-alkyle en C₁-C₂ et-alkyle en C₁-C₂-NR-alkyle en C₁-C₂-, dans lequel R représente un atome d'hydrogène ou un alkyle en C₁-C₆ ;
R^{b} et R^{b'} sont choisis indépendamment dans le groupe consistant en un atome d'hydrogène, un alkyle en C₁₋C₆, un alcényle en C₁₋C₆, un alkyle en C₁₋C₆, un alkyle en C₃₋C₆ ramifié, un cycloalkyle en C₃₋C₆ et un aryle ; et
R¹ représente :
- une fraction aliphatique ou hétéroaliphatique choisie dans le groupe constitué par un alkyle en C₁₋C₁₀, un alcényle en C₂₋C₁₀, un alcynyle en C₂₋C₁₀, un alkyle en C₁₋C₅-cycloalkyle en C₃₋C₆, (C₁-C_{y})-O-(C₁-Cₓ) et (C₁-C_{y})-S-(C₁-Cₓ), où y et x représentent chacun un nombre entier compris entre 0 et 7 et où y + x ≤ 7, chaque fragment étant éventuellement substitué par 1 à 3 substituants choisis indépendamment parmi un halogéno, un alkyle en C₁₋C₆, un cycloalkyle en C₃₋C₆, un phényle, un halogénoalkyle en C₁₋C₆, un alcoxy en C₁-C₆ et CH₂-O-alkyle en C₁₋C₆,
- un fragment aromatique choisi dans le groupe consistant en un phényle et un naphtyle, chacun éventuellement substitué par 1 à 3 substituants indépendamment choisis parmi un halogéno, un alkyle en C₁₋C₆, un alkyle en C₃₋C₆, un cycloalkyle en C₁₋C₆, un phényle, un halogénoalkyle en C₁ à C₆, un alcoxy en C₁ et un C₂-O-alkyle en C₁₋C₆, ou
- un fragment hétéroaromatique choisi dans le groupe consistant en thiényle, benzothiényle, furyle, benzopyrannyle, pyrole, indolyle, pyranne, pyrimidine, triazine, pyridyle, quinolyle, isoquinolyle, isoxazole, oxazole. pyrazole, thiazole, imidazole, triazole, oxadiazole, thiadiazole et tétrazole, chacun éventuellement substitué par 1 à 3 substituant indépendamment choisi parmi un halogéno, un alkyle en C₁₋C₆, un cycloalkyle en C -C₆, un phényle, un halogénoalkyle en C₁-C₅, un alcoxy en C₁ à C₆ et un CH₂-O-alkyle en C₁₋C₆.

2. Composé selon la revendication 1, dans lequel R^{a} et R^{a'} représentent tous deux un méthyle.

3. Composé selon la revendication 1, dans lequel l'un de R^{b} et R^{b'} représente un méthyle ou éthyle, de préférence un méthyle, et l'autre parmi R^{b} et R^{b'} représente un atome d'hydrogène.

4. Composé selon la revendication 1, dans lequel R^{b} et R^{b'} représentent tous deux un atome d'hydrogène.

5. Composé selon la revendication 1, dans lequel n = 1 ou 2, de préférence n = 1.

6. Composé selon la revendication 1 ou 2, dans lequel m = 1 ou 2, de préférence m = 1.

7. Composé selon l'une quelconque des revendications précédentes, dans lequel m = 1 et R¹ représente un alkyle en C₁ à C₁₀.

8. Composé selon l'une quelconque des revendications précédentes, dans lequel m = 1 ou 2 et R¹ représente un groupe phényle, éventuellement substitué par un ou deux substituants choisis dans le groupe constitué par un méthyle, un éthyle, un propyle, un t-butyle, un méthoxy, un éthoxy, un chloro, un fluoro et un trifluorométhyle.

9. Composé selon l'une quelconque des revendications précédentes, dans lequel m = 1 ou 2 et R¹ représente un groupe tel que défini dans la revendication 1, qui est également un hétéroaryle en C₅ ou C₆ comprenant un hétéroatome choisi parmi oxygène, azote et soufre, éventuellement substitué par un substituant choisi dans le groupe constitué par un méthyle, un éthyle, un propyle, un t-butyle, un méthoxy, un éthoxy, un chloro, un fluoro et un trifluorométhyle.

10. Composé selon la revendication 1, choisi dans le groupe constitué par CXP18.6-052 ; CXP 18.6-017 ; CXP 18.6-064 ; CXP 18.6-046 ; CXP 18.6-047 ; CXP 18.6-018 ; CXP 18.6-069 ; CXP 18.6-006 ; CXP 18.6-043 ; CXP 18.6-026 ; CXP14.18-019 ; CXP18.6-028 ; CXP18.6-042 ; CXP18.6-012 ; CXP14.18-038 ; CXP18.6-057 ; CXP18.6-019 ; CXP18.6-056 ; CXP 18.6-008 ; CXP18.6-027 ; CXP14.18-034 ; CXP18.6-013 ; CXP14.18-010 ; CXP14.18-008 ; CXP14.18-016 ; CXP14.18-028 ; CXP18.6-022 ; CXP14.18-037 ; CXP18.6-014; CXP14.18-035 ; CXP14.18-005 ; CXP14.18-014 ; CXP14.18-007 ; CXP14.18-012 ; CXP18.6-055 ; CXP14.18-020 ; CXP18.6-048 ; CXP18.6-007 ; CXP18.6-038 ; CXP18.6-009 ; CXP18.6-010, MMV689258, MMV689260 et des sels et esters pharmaceutiquement acceptables de ceux-ci, dans lequel lesdits composés sont définis comme suit :
| Compound | Structure |
|---|---|
| CXP18.6-052 | 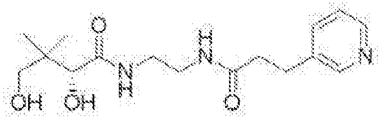 |
| CXP18.6-017 | 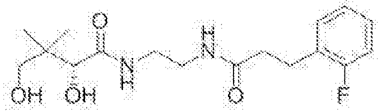 |
| CXP18.6-064 | 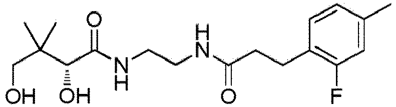 |
| CXP18.6-046 | 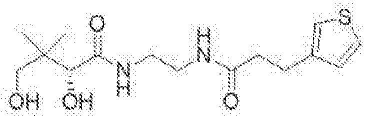 |
| CXP18.6-047 | 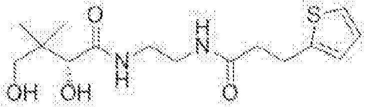 |
| CXP18.6-018 | 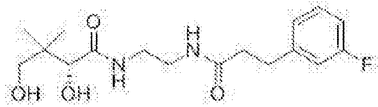 |
| CXP18.6-069 | 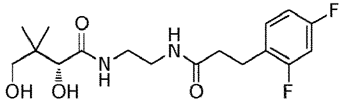 |
| CXP18.6-006 | 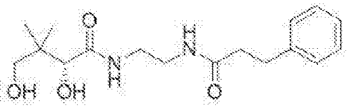 |
| CXP18.6-043 | 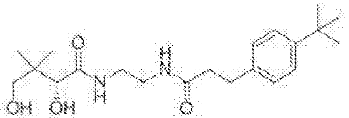 |
| CXP18.6-026 | 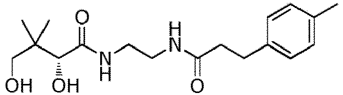 |
| CXP14.18-019 | 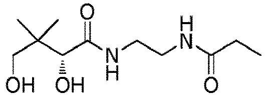 |
| CXP18.6-028 | 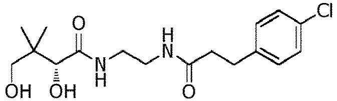 |
| CXP18.6-042 | 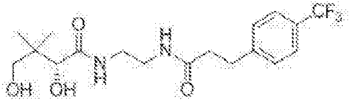 |
| CXP18.6-012 | 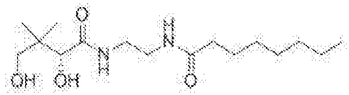 |
| CXP14.18-038 | 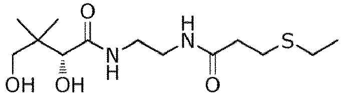 |
| CXP18.6-057 | 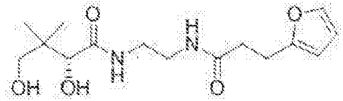 |
| CXP18.6-019 | 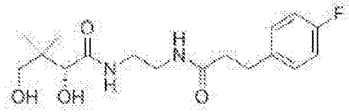 |
| CXP18.6-056 | 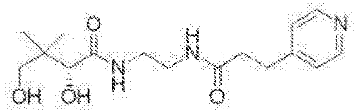 |
| CXP18.6-008 | 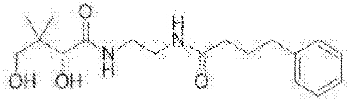 |
| CXP18.6-027 | 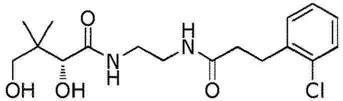 |
| CXP14.18-034 | 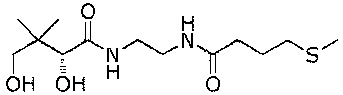 |
| CXP18.6-013 | 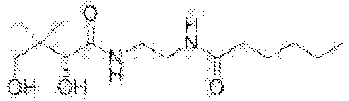 |
| CXP14.18-010 | 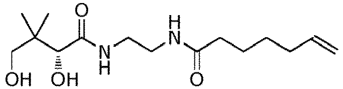 |
| CXP14.18-008 | 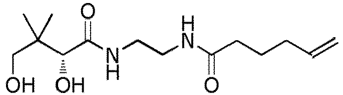 |
| CXP14.18-016 | 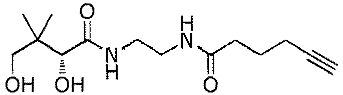 |
| CXP14.18-028 | 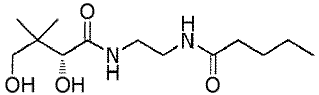 |
| CXP18.6-022 | 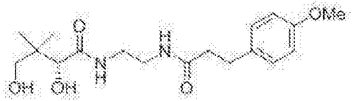 |
| CXP14.18-037 | 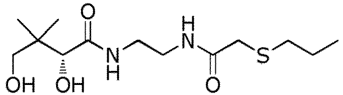 |
| CXP18.6-014 | 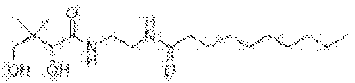 |
| CXP14.18-035 | 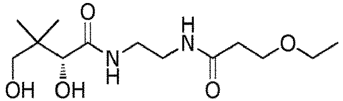 |
| CXP14.18-005 | 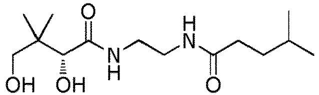 |
| CXP14.18-014 | 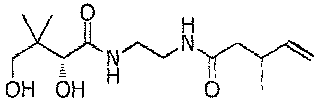 |
| CXP14.18-007 | 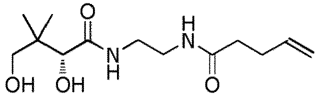 |
| CXP14.18-012 | 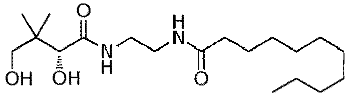 |
| CXP18.6-055 | 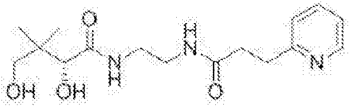 |
| CXP14.18-020 | 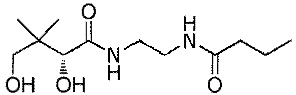 |
| CXP18.6-048 | 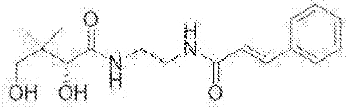 |
| CXP 18.6-007 | 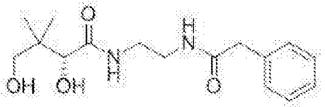 |
| CXP18.6-038 | 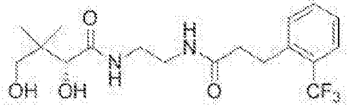 |
| CXP18.6-009 | 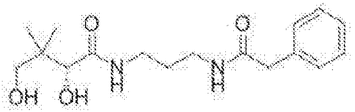 |
| CXP18.6-010 | 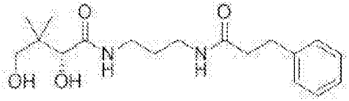 |
| MMV689258 | 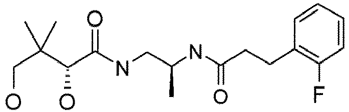 |
| MMV689260 | 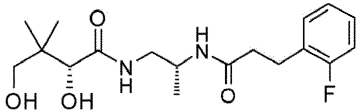 |

11. Composition pharmaceutique comprenant un composé tel que défini dans l'une quelconque des revendications 1 à 10 et un excipient pharmaceutiquement acceptable.

12. Composé tel que défini dans l'une quelconque des revendications 1 à 10, destiné à être utilisé dans une méthode de traitement ou de prévention d'une infection microbienne chez un sujet humain ou animal nécessitant un tel procédé, ladite méthode comprenant l'administration audit sujet d'un composé tel que défini dans des revendications 1 à 10.

13. Composé à utiliser selon la revendication 12, dans lequel ladite infection microbienne est choisie parmi une infection bactérienne, une infection fongique et une infection protozoaire.

14. Composé tel que défini dans l'une quelconque des revendications 1 à 10, destiné à être utilisé dans une méthode de traitement ou de prévention d'une maladie chez un sujet humain ou animal, ledit procédé comprenant l'administration audit sujet d'un composé tel que défini dans l'une quelconque des revendications 1 à 10, dans lequel ladite maladie est une maladie provoquée par des protozoaires, de préférence par des espèces de protozoaires choisies parmi le genre Plasmodium, Trypanosoma, Giardia, Cryptosporidium, Amoeba, Toxoplasma, Trichomonas ou Leishmania.

15. Composé tel que défini dans l'une quelconque des revendications 1 à 10, destiné à être utilisé dans une méthode de traitement ou de prévention d'une maladie chez un sujet humain ou animal, ladite comprenant l'administration audit sujet d'un composé tel que défini dans l'une quelconque des revendications 1 à 10, dans lequel ladite maladie est choisie dans le groupe consistant en paludisme, amibiase, giardiase, toxoplasmose, cryptosporidiose, trichomonase, maladie de Chagas, leishmaniose, maladie du sommeil, dysenterie, kératite à Acanthamoeba, méningo-encéphalite amibienne primaire.
